# EUROPEAN PATENT APPLICATION

(11) **EP 3 650 466 A1**
(43) Date of publication of application: **13.05.2020**
(21) Application number: 18382799.7
(22) Date of filing: 12.11.2018
(51) Int. Cl.: C07K 16/00, C12N 5/0781, C12N 5/0783, A61K 39/385, C07K 14/77

(54) **IN VITRO PRODUCTION OF HIGH AFFINITY MONOCLONAL ANTIBODIES**

(71) Applicant: Consejo Superior De Investigaciones Científicas, 28006 Madrid (ES)
(72) Inventor: MARTINEZ RIAÑO, Ana, 28049 Madrid (ES); ALARCON SANCHEZ, Balbino, 28049 Madrid (ES)
(74) Representative: Pons

(57) **Abstract**

The invention relates to a method for the *in vitro* generation of antigen-specific antibodies or cells producing thereof, said method comprising culturing B cells with an antigen-coated carrier for at least 3 days, wherein said antigen-coated carrier has a size between 0.5 µm and 20 µm, and co-culturing the B cells obtained from step a) with CD4+ T cells for at least 3 days. Thus, high-affinity class-switched immunoglobulins of clinical and diagnostic interest are produced *in vitro.*

## Description

### TECHNICAL FIELD OF THE INVENTION

The invention relates to the production of high affinity monoclonal antibodies by culturing B cells with an antigen-coated carrier for at least 3 days in the presence of CD4+ T cells. Thus, the invention relates to a method for selecting and generating high-affinity class-switched immunoglobulins of clinical and diagnostic interest *in vitro.* Consequently, the invention relates to the field of immunology.

### BACKGROUND ART

For an efficient protective humoral response to pathogen-derived protein antigens, B cells establish an intimate collaboration with antigen-specific helper T cells. To obtain T cell help, B cells have to recognize cognate antigen via their B cell antigen receptor (BCR), internalize it and present it once processed as MHC class II associated peptides. CD4 T cells that are able to recognize the processed antigen will become activated and express ligands of costimulatory receptors in B cells that, in turn, will initiate immunoglobulin class-switching and somatic hypermutation. These processes result in the selection of B cells bearing BCRs containing class-switched mature immunoglobulins of high affinity for antigen. This B-T cell cooperation takes place in germinal centers (GC) where B cells undergo iterative cycles of antigen recognition and presentation to T cells followed by very rapid cell proliferation and expansion. It is generally accepted that in GCs, B cells establish a fierce competition for antigen to gain T cell help resulting in the selection of B cells bearing BCRs with the highest affinity. The BCR can interact and be activated by soluble proteins although it is believed that, most frequently, B cells recognize and take up antigen deposited on the surface of antigen-presenting follicular dendritic cells. It has long been thought that only antigen-presenting cells of myeloid origin are able to phagocytose antigens, for B cells are incompetent to phagocytose particulate antigens. However, growing evidence suggests that B cells can also perform phagocytic functions. The capacity of B cells to phagocytose antigen was first described in early vertebrates, but lately it has also been demonstrated that murine B-1 B cell populations and human B cells can phagocytose bacteria. B cells receive help from a type of activated helper CD4 T cell known as T follicular helper cells (Tfh) which release important cytokines that stimulate B cell proliferation and modulate Ig class switching, including IL4 and IL21, and express ligands (CD40L, ICOS) for costimulatory receptors in B cells (CD40 and ICOSL). B cells integrate signaling emanating from their antigen-engaged BCR, from ligated CD40 and ICOSL and from cytokine receptors to undergo or continue their program of affinity maturation and Ig class switching. In this context, the BCR has a dual function, first as a provider of activation signals to the B cell and second as a mediator of antigen internalization, processing and presentation to T cells.

One of the caveats in studying the molecular processes that take place during B-T cell interaction in detail is to recreate GC *in vitro.* Different protocols consisting of mixtures of cytokines and the expression of CD40L in non-T cells have been used (Nojima et al., 2011, Nat Commun. 2:465). However, these procedures are not antigen-specific and have not allowed selecting for Ig class-switched B cells with increasing affinity for antigen.

The patent application US2016/0046907 describes methods to produce vaccines and antibodies, which methods including contacting follicular dendritic cells (FDC) with naive B-cells to mimic conditions in the CG *in vitro,* including methods of enhancing antibody production in hybridoma cells and compositions comprising product of the instant methods.

The patent application US2010/0184148 incorporates GCs into three-dimensional (3D) engineered tissue constructs (ETCs). In an embodiment, the GC has been incorporated in the design of an artificial immune system (AIS) to examine immune responses to vaccines and other compounds. Development of an *in vitro* GC adds functionality to an AIS, in that it enables generation of an *in vitro* human humoral response by human B lymphocytes, that is accurate and reproducible, without using human subjects. The invention also permits evaluation of, for example, vaccines, allergens, and immunogens, and activation of human B cells specific for a given antigen, which can then be used to generate human antibodies.

The patent application US2010/0323401 relates to an *in vitro* method of producing antigen specific B cells and antibodies that provides for the capture of an entire primary human antibody repertoire for any foreign antigen, allows for screening large numbers of immunogen/adjuvant combinations, and permits the isolation of human monoclonal antibodies on demand thereby obviating the need to immunized humans with the target antigen.

The international application WO2011/023705 relates to an *in vitro* method for the production of monoclonal antibodies, in particular, IgG type, making use of antigen presentation by dendritic cells.

Nevertheless, it is still felts that there is a great need for alternative methods to those disclosed in the state of the art for producing high-affinity class-switched immunoglobulins which recreates the *in vitro* germinal center response.

### DETAILED DESCRIPTION OF THE INVENTION

The inventors have developed an *in vitro* germinal center (GC) for producing high-affinity class-switched immunoglobulins. The inventors realized that when the antigen is bound to a particle having a size between 0.5 µm and 20 µm, said antigen is surprisingly phagocytosed through the B cell receptor (BCR), resulting in a stronger and more sustained BCR signal compared to stimulation with the soluble antigen (see Example), which in turn induce T cell and B cell proliferation as well as acquisition of B cell and T cell markers typical of a GC response. Still more surprisingly, the inventors realized that the presence of dendritic cells (DC) is no longer necessary for elicit a proper immune response. Thus, in the *in vitro* GC developed by the inventors, B cells recognized antigen through their BCR and this recognition has a dual effect: the activation of intracellular signaling pathways and antigen internalization for processing and presentation to CD4⁺ T cells. This allows the skilled person in the art to examine immune responses (especially humoral response) to vaccines, allergens, immunogens, immunomodulators, immunotherapies and other agents.

Based on this finding, the inventors have developed a method for producing high affinity monoclonal antibodies which specifically bind to an givenantigen.

### Method of the invention

As explained in the previous paragraph, the inventors have developed an *in vitro* germinal center (GC) for producing high-affinity class-switched immunoglobulins.

Thus, in an aspect, the present invention relates to method for the *in vitro* generation of high affinity antigen-specific antibodies or cells producing thereof, hereinafter "method of the invention", said method comprising:
a) culturing B cells with an antigen-coated carrier for at least 3 days, wherein said antigen-coated carrier has a size between 0.5 µm and 20 µm, and
b) co-culturing the B cells obtained from step a) with CD4+ T cells for at least 3 days.

This method recreates the *in vitro* GG response.

As used herein, the term "high affinity antigen-specific antibodies" (interchangeably used in plural form) refers to an immunoglobulin molecule capable of specific binding to a target, such as a polypeptide, carbohydrate, polynucleotide, lipid, etc., in a monovalent fashion with an affinity constant (K_{D}) lower than 10 µM. An antibody includes an antibody of any class, such as IgD, IgE, IgG, IgA, or IgM (or sub-class thereof), and the antibody need not be of any particular class. Depending on the antibody amino acid sequence of the constant domain of its heavy chains, immunoglobulins can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgA1 and IgA2. The heavy-chain constant domains that correspond to the different classes of immunoglobulins are called alpha, delta, epsilon, gamma, and mu, respectively. The subunit structures and three-dimensional configurations of different classes of immunoglobulins are well known.

As used herein, the term "cells producing antigen-specific antibodies" refers to differentiated B cells capable of secreting soluble antibodies in the surrounding medium.

As it is widely known, antibodies can occur in two physical forms, a soluble form that is secreted from the cell to be free in the blood plasma, and a membrane-bound form that is attached to the surface of a B cell and is referred to as the B-cell receptor (BCR). The BCR is found only on the surface of B cells and facilitates the activation of these cells and their subsequent differentiation into either antibody factories called plasma cells or memory B cells that will survive in the body and remember that same antigen so the B cells can respond faster upon future exposure. In most cases, interaction of the B cell with a T helper cell is necessary to produce full activation of the B cell and, therefore, antibody generation following antigen binding.

In a first step, the method of the invention comprises culturing B cells with an antigen-coated carrier for at least 3 days, wherein said antigen-coated carrier has a size between 0.5 µm and 20 µm.

The term "B cell", also known as B lymphocyte, refers to a type of white blood cell of the lymphocyte subtype. They function in the humoral immunity component of the adaptive immune system by secreting antibodies. B cells express B cell receptors (BCRs) on their cell membrane, which allow the B cell to bind a specific antigen against which it will initiate the antibody response. Examples of B cells include, without limiting to, plasmablast, plasma cell, memory B cell, follicular (FO) B cell (also known as a B-2 cell), marginal zone (MZ) B cell and regulatory B (Breg) cell. Any B cell can be used in the composition of the present invention. Nevertheless, in a particular embodiment, the B-cells from step a) of the method of the invention are naive B cells expressing non-class-switched immunoglobulins (IgM and/or IgD) or memory B cells expressing class-switched immunoglobulins (IgGs, IgA, IgE).

The term "naive B cell" refers to a B cell that has not been exposed to an antigen. Once exposed to an antigen, the naive B cell either becomes a memory B cell or a plasma cell that secretes antibodies specific to the antigen that was originally bound.

The term "memory B cell" refers to B cell sub-type formed within GCs following primary infection or immunization. They generate an antibody-mediated immune response in the case of re-infection or re-immunization.

In another particular embodiment, the B cells are follicular B cells comprising low expression levels of CD21, high expression levels of CD23, and CD43 negative. Methods for analyzing the expression level of cell surface markers are widely known in the state of the art. For example, the level of expression of each marker may be the analyzed by fluorescence-activated cell sorter, being the level of expression the geometric mean fluorescence intensity (MFI) of sample cells. Low CD21 and high CD23 expression are based on the comparison of how these markers are expressed in the accompanying splenic marginal zone B cells (CD21^{high} CD23^{low}). A significant change in expression was defined as a fold change of greater than two with a P value < 0.05.

The B cells of the present invention may be isolated from different sources. Examples of sources include, without limiting to, bone marrow, lymph nodes, blood, and peripheral blood mononuclear cell (PBMC). Likewise, B cells coming from any animal may be used in the composition of the invention. In a preferred embodiment, the B cell comes from a mammal, for example, a mouse. In a more preferred embodiment, the B cell comes from a primate, preferably, a human. Thus, in a particular embodiment of the invention, the B cell is a human B cell.

As used herein, the term "antigen-coated carrier" refers to a molecule comprising one or more epitopes (either linear, conformational or both) that elicit an immunological response and having a size between 0.5 and 20 µm. In a particular embodiment, the size of the antigen-coated carrier is 1, 2, 3, 4, 5, 6, 7, 8, 9, 10, 11, 12, 13, 14, 15, 16, 17, 18, or 19 µm. The term is used interchangeably with the term "immunogen". Normally, a B-cell epitope will include at least about 5 amino acids but can be as small as 3-4 amino acids.. The term "antigen" denotes both subunit antigens, i.e., antigens which are separate and discrete from a whole organism with which the antigen is associated in nature, as well as killed, attenuated or inactivated bacteria, viruses, fungi, parasites or other microbes. Antibodies such as anti-idiotype antibodies, or fragments thereof, and synthetic peptide mimotopes, which can mimic an antigen or antigenic determinant, are also captured under the definition of antigen as used herein. Similarly, an oligonucleotide or polynucleotide which expresses an antigen or antigenic determinant in vivo, such as in gene therapy and DNA immunization applications, is also included in the definition of antigen herein.

Furthermore, for purposes of the present invention, an "antigen" refers to a protein which includes modifications, such as deletions, additions and substitutions (generally conservative in nature), to the native sequence, so long as the protein maintains the ability to elicit an immunological response. These modifications may be deliberate, as through site-directed mutagenesis, or may be accidental, such as through mutations of hosts which produce the antigens.

An antigen can be derived from multiple sources. The selection of an immunogen against which antibodies are to be raised will, of course, depend upon clinical interest. Some clinically significant immunogens include bacterial antigens, viral antigens, toxins, blood group antigens, antigens on lymphoid cells, myosin and tumor antigens such as cell-associated antigens and tumor cell secreted products.

Some smaller antigens, such as haptens, may also be polymerized to increase immunogenicity. Linking agents useful in the coupling of smaller antigens to carriers include carbodiimides; glutaraldehyde; N-N-carbonyldiimidazole; 1-hydroxybenzotriazole monohydrate; N-hydroxy succinimide; N-trifluoroacetylimidazole; cyanogen bromide; and bis-diazotized benzidine.

The antigen may be either a foreign antigen or an endogenous antigen. As used herein, "foreign antigen" refers to a protein or fragment thereof, which is foreign to the recipient animal cell, but not limited to, a viral protein, a parasite protein, or an immunoregulatory agent. The term "endogenous antigen" is used herein to refer to a protein or part thereof that is naturally present in the recipient animal cell, such as a cellular protein, or immunoregulatory agent. These pathogens can be infectious in humans, domestic animals or wild animal hosts. The foreign antigen can be any molecule that is expressed by any pathogen prior to or during entry into, colonization of, or replication in their animal host. In a particular embodiment, the antigen is derived from a pathogen, preferably selected from the group consisting of virus, bacteria, yeast and protozoa.
As used herein, the term "pathogen" refers to anything that can produce disease. Examples of pathogens included, without limiting to, virus, bacteria, yeast and protozoa.

Viral pathogens, from which viral antigens are derived may include, but are not limited to, Orthomyxoviruses, such as influenza virus; Retroviruses, such as RSV, HTLV-1 and HTLV-II; Herpes viruses, such as EBV, CMV or herpes simplex virus; Lentiviruses, such as HIV-1 and HIV-2; Rhabdoviruses, such as rabies; Picornoviruses, such as Poliovirus; Poxviruses, such as vaccinia; Rotavirus; Rhinovirus and Parvoviruses, such as adeno-associated virus 1 (AAV-1).

Examples of viral antigens include, but are not limited to, the human immunodeficiency virus antigens Nef, Gag, Env, Tat, Rev, Pol and T cell and B cell epitopes of gp120, such as CD4, fragment thereof or mimetics thereof; chimeric polypeptides including receptor-ligand pairs including Env proteins and virus antigens, such as VP4 and VP7; influenza virus antigens, such as hemagglutinin; nucleoprotein; herpes simplex virus antigens; and toxins such as botulism, spider toxins; hepatitis B surface antigen; other toxins including avian viruses. In a particular embodiment of the invention, the antigen is the Env recombinant protein of HIV.

The bacterial pathogens, from which the bacterial antigens are derived, include but are not limited to, Mycobacterium spp., Helicobacter pylori, Salmonella spp., Shigella spp., E. coli, Rickettsia spp., Listeria spp., Legionella pneumoniae, Pseudomonas spp., Vibrio spp., and Borellia burgdorferi. Examples of protective antigens of bacterial pathogens include, without limiting to, the somatic antigens of enterotoxigenic E. coli, such as the CFA/I fimbrial antigen and the nontoxic B-subunit of the heat-labile toxin; pertactin of Bordetella pertussis, adenylate cyclase-hemolysin of B. pertussis, fragment C of tetanus toxin of Clostridium tetani, OspA of Borellia burgdorferi, protective paracrystalline-surface-layer proteins of Rickettsia prowazekii and Rickettsia typhi, the listeriolysin (also known as "Llo" and "Hly") and/or the superoxide dismutase (also known as "SOD" and "p60") of Listeria monocytogenes; the urease of Helicobacter pylori, and the receptor-binding domain of lethal toxin and/or the protective antigen of Bacillus anthrax. Example of antigens from biological weapons or pathogens include, but are not limited to, smallpox, anthrax, tularemia, plague, listeria, brucellosis, hepatitis, vaccinia, mycobacteria, coxsackievirus, tuberculosis, malaria, erhlichosis and bacterial meningitis. Examples of non-peptide bacterial antigens include, but are not limited to: LPS, foreign polysaccharides and unmethylated CpG DNA.

The parasitic pathogens, from which the parasitic antigens are derived, include but are not limited to, Plasmodium spp., such as Plasmodium falciparum; Trypanosome spp., such as Trypanosoma cruzi; Giardia spp., such as Giardia intestinalis; Boophilus spp.; Babesia spp., such as Babesia microti; Entamoeba spp., such as Entamoeba histolytica; Eimeria spp., such as Eimeria maxima; Leishmania spp., Schistosome spp., such as Schistosoma mansoni; Brugia spp., such as Brugia malayi; Fascida spp., such as Fasciola hepatica; Dirofilaria spp., such as Dirofilaria immitis; Wuchereria spp., such as Wuchereria bancrofti; and Onchocerea spp; such as Onchocerca volvulus. Examples of parasite antigens include, but are not limited to, the pre-erythrocytic stage antigens of Plasmodium spp., such as the circumsporozoite antigen of P. falciparum; P vivax; the liver stage antigens of Plasmodium spp., such as the liver stage antigen 1; the merozoite stage antigens of Plasmodium spp., such as the merozoite surface antigen-1 (also referred to as MSA-1 or MSP-1); the surface antigens of Entamoeba histolytic, such as the galactose specific lectin or the serine rich Entamoeba histolytic protein (also referred to as SREHP); the surface proteins of Leishmania spp. (also referred to as gp63); such as 63 kDa glycoprotein (gp63) of Leishmania major or the 46 kDa glycoprotein (gp46) of Leishmania major; paramyosin of Brugia malayi; the triose-phosphate isomerase of Schistosoma mansoni; the secreted globin-like protein of Trichostrongylus colubriformis; the glutathione-S-transferases of Fasciola hepatica; Schistosoma bovis; S. japonicum; and KLH of Schistosoma bovis and S. japonicum.

Examples of pathogenic yeast include, without limiting to, *Candida albicans, Candida tropicalis and Trichophyton mentagrophytes, Trichophyton interdigitale, Trichophyton rubrum, and Trichophyton yaoundei.*

Examples of fungal pathogens include, without limiting to, *Candida spp. including C. albicans, C. tropicalis, C. kefyr, C. krusei and C. galbrata; Aspergillus spp. including A. fumigatus and A. flavus; Cryptococcus neoformans; Blastomyces spp. including Blastomyces dermatitidis; Pneumocystis carinii; Coccidioides immitis; Basidiobolus ranarum; Conidiobolus spp.; Histoplasma capsulatum; Rhizopus spp. including R. oryzae and R. microsporus; Cunninghamelia spp.; Rhizomucor spp.; Paracoccidioides brasiliensis; Pseudallescheria boydii; Rhinosporidium seeberi; and Sporothrix schenckii.*

Examples of protozoa pathogens include, without limiting to, *Trypanosoma cruzi, Trypanosoma brucei, Leishmania spp., Naegleria fowleri, Giardia intestinalis, Acanthamoeba castellanii, Balamuthia mandrillaris, Entamoeba histolytica, Blastocystis hominis, Babesia microti, Cryptosporidium parvum, Cyclospora cayetanensis, Plasmodium spp. and Toxoplasma gondii.*

Examples of tumor specific antigens include prostate specific antigen (PSA), TAG-72 and CEA; human tyrosinase; tyrosinase-related protein (also referred to as TRP); and tumor-specific peptide antigens.

Examples of autoimmune antigens include IAS β chain, which is useful in therapeutic vaccines against autoimmune encephalomyelitis; glutamic acid decarboxylase, which is useful in therapeutic vaccines against insulin-dependent type 1 diabetes; thyrotropin receptor (TSHr), which is useful in therapeutic vaccines against Grave's disease and tyrosinase-related protein 1, which is useful in therapeutic vaccines against vitiligo.

Endogenous antigen, which may be any cellular protein or immunoregulatory agent, or parts thereof, expressed in the blood donor are also applicable to the present invention including, but not limited to, tumor, transplantation and autoimmune antigens, or fragments and derivatives of tumor, transplantation and autoimmune antigens thereof. Another endogenous antigen that may be of clinical interest is any of the antigens of the human blood group systems including, but without limiting to, AB0, MNS, P, Rh, Lutheran, Kell, Lewis, Duffy, Kidd, Diego, Yt, XG, Scianna, Dombrock, Colton, Landsteiner-Wiener, Chido, Hh, XK, Gerbich, Cromer, Knops, Indian, Ok, Raph, JMH, li, Globoside, GIL, Rh-associated glycoprotein, Forssman, Langereis, Junior, Vel, CD59 and Augustine.

In a particular embodiment of the method of the method, the antigen is selected from the group consisting of an hapten, peptide and protein or fragments of any thereof. In a more particular embodiment, the protein is a glycoprotein or a lipoprotein.

As used herein, the term "hapten" refers to minute molecules that elicit an immune response only when attached to a large carrier such as a protein; the carrier may be one that also does not elicit an immune response by itself. Once the body has generated antibodies to a hapten-carrier adduct, the small-molecule hapten may also be able to bind to the antibody, but it will usually not initiate an immune response; usually only the hapten-carrier adduct can do this. Sometimes the small-molecule hapten can even block immune response to the hapten-carrier adduct by preventing the adduct from binding to the antibody, a process called *hapten inhibition.* Haptens which may be employed in the present invention include, without limiting to steroids such as estrone, estradiol, testosterone, pregnanediol and progesterone; vitamins such as B12, biotin and folic acid; triodothyronine, thyroxine, histamine, serotonine, digoxin, prostaglandins, adrenalin, noradrenalin, morphine, vegetable hormones, antibiotics such as penicillin, bacterial or chemical toxins, chemical compounds such as insecticides, bacterial or viral polysaccharides, etc.

Extended linking groups are groups that will bind the hapten to the macromolecule or macromolecule fragment in such a way that the hapten is still able to undergo binding by an anti-hapten. Extended linking groups useful in the present invention include succinylated polylysine, dextran, polyethyleneglycol, and preferentially a polyamido ether extending group. These extended linking groups may be used separately or in combination to obtain extended linking groups of varying lengths and binding properties. Extended linking groups are preferred for use with serum samples and especially lipemic serum samples. Evidently, there are interfering substances in serum samples, the interference from which is overcome by the extended linking group. Where an extended linking group is not needed, a hapten such as biotin, without an extended binding group is bound to a functional group on a membrane or to a functional group on a protein which can be disbursed on the membrane. Various methods exist which may be employed to bind the extended linking group to a macromolecule or fragment. For example, to facilitate this binding the extended linking group may be attached to macromolecule-reactive groups such as active ester groups, amino groups, sulfhydryl groups, carbohydrate groups, azido groups or carboxy groups.

As used herein, the term "peptide" generally refers to a linear chain of around 2 to 50 amino acid residues joined together with peptide bonds. It will be understood that the terms "peptide bond" is known to the person skilled in the art. As used herein, an "amino acid residue" refers to any naturally occurring amino acid, any amino acid derivative or any amino acid mimic known in the art.

As used herein, the term "protein" refers to a polymer of more than 50 amino acid residues linked together by peptide bonds. The term, as used herein, refers to proteins and polypeptides of any size, structure, or function. A protein may be naturally occurring, recombinant, or synthetic, or any combination of these. A protein may be a single molecule or may be a multi-molecular complex. The term protein may also apply to amino acid polymers in which one or more amino acid residues is an artificial chemical analogue of a corresponding naturally occurring amino acid. An amino acid polymer in which one or more amino acid residues is an "unnatural" amino acid, not corresponding to any naturally occurring amino acid, is also encompassed by the use of the term "protein" herein.

As used herein, the term "glycoprotein" or "glycopeptide" refers to protein or peptide, respectively, comprising oligosaccharide chains (glycans) covalently attached to amino acid side-chains. As used herein, the term "lipoprotein" refers to a biochemical assembly comprising protein and lipid whose purpose is to transport hydrophobic lipid (a.k.a. fat) molecules in water, as in blood or extracellular fluid. Examples include, without limited to, the plasma lipoprotein particles classified as HDL, LDL, IDL, VLDL and ULDL (a.k.a. chylomicrons) lipoproteins, according to density / size (an inverse relationship), compared with the surrounding plasma water.

In the context of the present invention, the term antigen also encompasses fragments of hapten, peptide, protein, glycoprotein or lipoprotein, as long as these fragments show the capacity of eliciting an immune response when attached to a large carrier.

In a particular embodiment, the antigen-coated carrier from step a) is an aggregate of small particles or a magnetic particle, preferably, the magnetic particle is a paramagnetic particle.

The terms "particle" and "microparticle" are equivalent and can be used interchangeably throughout the present description. If the particle is a sphere, the maximum particle size corresponds to the diameter of the particle. The particle can be of any shape, such as irregular, round (e.g. beads), oval, etc. Particle size is readily determined by techniques well known in the art, such as photon correlation spectroscopy, laser diffractometry and/or scanning electron microscopy.

Particles for use herein can be formed from materials that are sterilizable, non-toxic and biodegradable. Such materials include, without limitation, poly(a-hydroxy acid), polyhydroxybutyric acid, polycaprolactone, polyorthoester, polyanhydride, PACA, and polycyanoacrylate. Particles for use with the present invention are derived from a poly(α-hydroxy acid), in particular, from a poly(lactide) ("PLA") or a copolymer of D,L-lactide and glycolide or glycolic acid, such as a poly(D,L-lactide-co-glycolide) ("PLG" or "PLGA"), or a copolymer of D,L-lactide and caprolactone. The particles may be derived from any of various polymeric starting materials which have a variety of molecular weights and, in the case of the copolymers such as PLG, a variety of lactide:glycolide ratios. For example, a 50:50 PLG polymer, containing 50% D,L-lactide and 50% glycolide, will provide a fast resorbing copolymer while 75:25 PLG degrades more slowly, and 85:15 and 90:10, even more slowly, due to the increased lactide component. It is readily apparent that a suitable ratio of lactide:glycolide is easily determined by one of skill in the art based on the nature of the antigen and disorder in question. PLG copolymers with varying lactide:glycolide ratios and molecular weights are readily available commercially from a number of sources including from Boehringer Ingelheim, Germany and Birmingham Polymers, Inc., Birmingham, Ala. These polymers can also be synthesized by simple polycondensation of the lactic acid component using techniques well known in the art. In a particular embodiment, the material of the particle is carboxylated latex

The particles can be formed with detergents, such as cationic, anionic, or nonionic detergents, which detergents may be used in combination. The term "detergent" as used herein includes surfactants and emulsion stabilizers. Anionic detergents include, but are not limited to, SDS, SLS, sulphated fatty alcohols, and the like. Cationic detergents include, but are not limited to, cetrimide (CTAB), benzalkonium chloride, DDA (dimethyl dioctodecyl ammonium bromide), DOTAP, and the like. Nonionic detergents include, but are not limited to, sorbitan esters, polysorbates, polyoxyethylated glycol monoethers, polyoxyethylated alkyl phenols, poloxamers, and the like.

Magnetic particles are also encompassed within the present invention in order to isolate the b-cell which has phagocytosed the antigen. Thus, in another particular embodiment, the particle is a magnetic particle or a paramagnetic particle. These particles are magnetic due to the inclusion of a form of magnetic metal. Example of magnetic metals include, without limiting to, iron, nickel, cobalt.

The term "magnetic particle" or "magnetizable particle" is defined herein as any particle dispersible or suspendible in aqueous media without significant gravitational settling and is separable from suspension by application of a magnetic field, which particle comprises a magnetic metal oxide core generally surrounded by an adsorptively or covalently bound sheath or coat bearing organic functionalities to which antigen can be attached. The terms "magnetizable aggregate" and "magnetizable particle" are equivalent and can be used interchangeably.

"Magnetic" and "magnetizable" as used herein encompass materials that may or may not be permanently magnetic, including superparamagnetic, ferromagnetic, and paramagnetic materials. Superparamagnetic materials have high magnetic susceptibility and thus become magnetized in the presence of a magnetic field, but lose their magnetization when the magnetic field is removed. Ferromagnetic materials are strongly susceptible to magnetic fields and remain magnetized when the field is removed. Paramagnetic materials have only a weak magnetic susceptibility and when the field is removed quickly lose their weak magnetization.

Magnetic particles of the invention may be made by preparing a solution of divalent (Fe2+) and trivalent (Fe3+) iron salts in acid and treating the resulting mixture with ammonium hydroxide to form a slurry of magnetite. Magnetite (Fe₃O₄, black mineral form of iron oxide crystallizing in the cubic system) prepared in this manner consists of aggregates of small crystallites and is magnetizable. Preferably, the crystallites of the magnetizable transition metal oxides have a particle size of about 3 nm to about 25 nm. In other embodiments, the magnetizable particles are made from a magnetizable transition metal oxide by a similar process. The resulting slurry of magnetite or transition metal oxide can be converted into colloidal aggregates or magnetizable aggregates of magnetizable iron oxide by treatment with either acid (perchloric acid, nitric acid, or a similar non-complexing acid), a solution of a ferric salt, such as a ferric nitrate solution, excess ferric ion (in the presence of a non-complexing counterion) or base (tetramethylammonium hydroxide or a similar non-complexing base). These treatments result in depletion of ferrous ion from the magnetite by either ion exchange or oxidation, and this depletion of ferrous ion is an important part of the formation of the colloid or magnetizable aggregates.

The particle may show an adsorbent surface. The adsorption of the antigens to the surface of the adsorbent particles occurs via any bonding-interaction mechanism, including, but not limited to, ionic bonding, hydrogen bonding, covalent bonding, Van der Waals bonding, and bonding through hydrophilic/hydrophobic interactions. Those of ordinary skill in the art may readily select detergents appropriate for the type of macromolecule to be adsorbed. In a particular embodiment, the antigen is covalently bound to the particle. In the context of the present invention, particles can be couple to one, two or more different antigens.

Particles manufactured in the presence of charged detergents, such as anionic or cationic detergents, may yield particles with a surface having a net negative or a net positive charge, which can adsorb a wide variety of molecules. For example, microparticles manufactured with anionic detergents, such as sodium dodecyl sulfate (SDS), i.e. SDS-PLG particles, adsorb positively charged antigens, such as proteins. Similarly, particles manufactured with cationic detergents, such as hexadecyltrimethylammonium bromide (CTAB), i.e. CTAB-PLG particles, adsorb negatively charged macromolecules, such as DNA. Where the macromolecules to be adsorbed have regions of positive and negative charge, either cationic or anionic detergents may be appropriate.

The particles are prepared using any of several methods well known in the art. For example, double emulsion/solvent evaporation techniques can be used herein to make the particles. These techniques involve the formation of a primary emulsion consisting of droplets of polymer solution, which is subsequently mixed with a continuous aqueous phase containing a particle stabilizer/surfactant. Alternatively, a water-in-oil-in-water (w/o/w) solvent evaporation system can be used to form the particles. In this technique, the particular polymer is combined with an organic solvent, such as ethyl acetate, dimethylchloride (also called methylene chloride and dichloromethane), acetonitrile, acetone, chloroform, and the like. The polymer will be provided in about a 1-30%, preferably about a 2-15%, more preferably about a 3-10% and most preferably, about a 4% solution, in organic solvent. The polymer solution is emulsified using e.g., a homogenizer. The emulsion is then optionally combined with a larger volume of an aqueous solution of an emulsion stabilizer such as polyvinyl alcohol (PVA), polyvinyl pyrrolidone, and a cationic, anionic, or nonionic detergent. The emulsion may be combined with more than one emulsion stabilizer and/or detergent, e.g., a combination of PVA and a detergent. Certain antigens may adsorb more readily to particles having a combination of stabilizers and/or detergents. Where an emulsion stabilizer is used, it is typically provided in about a 2-15% solution, more typically about a 4-10% solution. Generally, a weight to weight detergent to polymer ratio in the range of from about 0.00001:1 to about 0.1:1 will be used, more preferably from about 0.0001:1 to about 0.01:1, more preferably from about 0.001:1 to about 0.01:1, and even more preferably from about 0.005:1 to about 0.01:1. The mixture is then homogenized to produce a stable w/o/w double emulsion. Organic solvents are then evaporated.

The formulation parameters can be manipulated to allow the preparation of a wide range of particle size. For example, reduced agitation results in larger particles, as does an increase in internal phase volume. Small particles are produced by low aqueous phase volumes with high concentrations of emulsion stabilizers.

Particles can also be formed using spray-drying and coacervation; air-suspension coating techniques, such as pan coating and Wurster coating; and ionic gelation.

Particle size can be determined by, e.g., laser light scattering, using for example, a spectrometer incorporating a helium-neon laser. Generally, particle size is determined at room temperature and involves multiple analyses of the sample in question (e.g., 5-10 times) to yield an average value for the particle diameter. Particle size is also readily determined using scanning electron microscopy (SEM).

Following preparation, particles can be stored as is or freeze-dried for future use. In order to adsorb antigens to the particles, the particle preparation is simply mixed with the antigen of interest and the resulting formulation can again be lyophilized prior to use. Generally, antigens are added to the particles to yield particles with adsorbed antigens having a weight to weight ratio of from about 0.0001:1 to 0.25:1 antigen to particles, preferably, 0.001:1 to 0.1, more preferably 0.01 to 0.05. Antigen content of the particles can be determined using standard techniques.

The term "immunological response" to an antigen refers to the development in a subject of a humoral and/or a cellular immune response to the antigen. For purposes of the present invention, a "humoral immune response" refers to an immune response mediated by antibody molecules, while a "cellular immune response" is one mediated by T-lymphocytes and/or other white blood cells. A "cellular immune response" also refers to the production of cytokines, chemokines and other such molecules produced by activated T-cells and/or other white blood cells, including those derived from CD4+ and CD8+ T-cells. The ability of a particular antigen to stimulate a cell-mediated immunological response may be determined by a number of assays, such as by lymphoproliferation (lymphocyte activation) assays, CTL cytotoxic cell assays, or by assaying for T-lymphocytes specific for the antigen in a sensitized subject. Such assays are well known in the art.

Thus, an immunological response as used herein may be one which stimulates the production of CTLs, and/or the production or activation of helper T-cells. The antigen of interest may also elicit an antibody-mediated immune response. Hence, an immunological response may include one or more of the following effects: the production of antibodies by, e.g., but not limited to B-cells; and/or the activation of suppressor T-cells and/or [gamma][delta] T-cells directed specifically to an antigen or antigens. These responses may serve to neutralize infectivity, and/or mediate antibody-complement, or antibody dependent cell cytotoxicity (ADCC) to provide protection to an immunized host. Such responses can be determined using standard immunoassays and neutralization assays, well known in the art.

The culture of the B cells with the antigen-coated carrier having a size of less than 20 µm can be carried out in any culture vessel or culture apparatus conventionally used in animal cell culture. Although Petri dishes, T-flasks and spinner flasks used on a laboratory scale, culture apparatuses equipped with cell separator using filters, gravity, centrifugal force used in high-density culturing of suspended cells, culturing apparatuses using harboring carriers such as micro-carriers or hollow fibers that are used mainly for high-density culture of adhesive cells, or bioreactors for industrial production can be used, the vessels or apparatuses are not limited to these examples.

Any medium ordinary used in culturing of animal cells may be used for the basal medium. Although either medium containing serum or that not containing serum may be used, serum-free media, which contain insulin, transferrin instead of serum, are preferable. Protein-free media are the most preferable. Recipes for growth media can vary in pH, glucose concentration, growth factors, and the presence of other nutrients.

Cells are grown and maintained at an appropriate temperature and gas mixture in a cell incubator for at least 3 days. In some embodiments, B cells are exposed to an antigen-coated carrier for about 3 to about 21 days. In particular embodiments, B cells are exposed to an antigen-coated carrier for about 7 to about 14 days. In a particular embodiment, B cells are exposed to an antigen-coated carrier for about 10 days. During exposure of B cells to an antigen-coated carrier, culture media can be removed and replaced with an equal amount of fresh media (e.g., the reaction media) as needed. For example, half the volume of media can be replaced at Day 4 and/or all of the media can be replaced with fresh media at Day 7. In exemplary embodiments, the viability of the B cells remains >50% after 10 days in tissue culture in the presence of the antigen. The culturing temperature is preferably the temperature optimal for growth. If the cells are derived from homothermal animals, a temperature of 36-38° C is common, while a temperature of 37° C is the most common.

During culture, there is a direct contact between the B cell and the antigen-coated carrier so that they are in immediate proximity or association with each other. B cells provided in a suitable cell culture media can be contacted with an antigen by adding (e.g., pipetting) the antigen directly to B cells suspended in the media. In other embodiments, B cells can be re-suspended in a fusion reaction media, the fusion reaction media including a suitable cell culture media and an effective amount of antigen.

The amount and concentration of antigen administered to the B cells is the amount and at a concentration sufficient to stimulate the B cells to generate antibodies specific to the antigen. The amount and concentration of the antigen can vary depending on a variety of factors, such as the immunogenicity of the antigen. In some embodiments, the amount of antigen administered to the B cells is substantially less than what would be required to stimulate antibody formation in a conventional monoclonal antibody preparation system. For example, in some embodiments, the amount of antigen administered can be about 50% less, about 75% less, or about 90% less than that typically administered to a live animal immunized as part of a monoclonal antibody production protocol.

Additionally an adjuvant or combination of adjuvants may be included in the culture medium to enhance the immune response. Examples of adjuvants have been previously described in the present disclosure. Notably, the adjuvant may be chosen to provide for not only the production of primary antibodies IgM but also other types such as IgG and IgA. In a particular embodiment, the adjuvant is alum.

A particular feature of the present invention refers to the absence in the culture of step a) of cells with antigen presenting capacity other than the B cells. Thus, in a particular embodiment of the method of the invention, method is further characterized by the absence in the culture of step a) of cells with antigen presenting capacity other than the B cells. Examples of cells with antigen presenting capacity other than the B cells include, without limiting dendritic cells.

The term "dendritic cells" refers to antigen-presenting cells (also known as accessory cells) of the mammalian immune system, whose main function is to process antigen material and present it on the cell surface to the T cells of the immune system. The dendritic cells can be obtained by any method known in the state in the art. Information about how to isolate and generate human dendritic cells can be found, for example, in Nair, S. et al. 2012, Curr Protoc Immunol. 0 7: Unit7.32.

After culturing B cells with an antigen-coated carrier for at least 3 days, the method of the invention comprises co-culturing the B cells obtained from step a) with CD4+ T cells for at least 3 days.

As used herein, the term "CD4+ T cell" or "*helper* T cell" (Tₕ cell) refers to a type of T cell which, when activated to become an effector cell, activates B cells to secrete antibodies and macrophages to destroy ingested microbes. In a particular embodiment of the present invention, the CD4+ T cells are naive CD62L+, CD4+ T cells or memory CD44+, CD4+ T cells. The CD4+ T cells may be isolated from different sources including, but without limiting to, thymus, lymph nodes, blood and peripheral blood mononuclear cell. Additionally, as B cells, helper T cells coming from any animal may be used in the composition of the invention. In a preferred embodiment, the helper T cell comes from a mammal, for example, a mouse. In a more preferred embodiment, the T cell comes from a primate, preferably, a human.

In step b) of the method of the invention any rate of B cells to CD4+T cells can be used. Nevertheless, in a particular embodiment, the ratio of B cells to CD4+T cells is 1:1.

Steps a) and b) from the method of the invention can be carried out two separate steps, i.e. first is carried out step a) and next step b), but, in a particular embodiment, the steps a) and b) are simultaneously executed.

As a consequence of putting into practice the method of the invention, high affinity class switched antibodies are produced. Thus, in a particular embodiment of the method of the invention, the method results in the production of high affinity class switched antibodies, wherein high affinity antibodies are characterized by binding to their antigen with a dissociation constant (K_{D}) of 10⁻⁵ to 10⁻¹² moles/liter or less.

As used herein, the term "dissociation constant" or "K_{D}" refers to the strength of binding between two components A and B. In the context of antibody-ligand binding, component A is the antibody and B is the ligand or antigen. This constant verifies how easy it is to separate the complex AB (dissociation). If a high concentration of A and B reactant is required to form the complex AB, then it also shows that the binding strength is low. The value of dissociation constant Kd would be higher as more of A and B are required to form complex AB. If a low concentration of A and B reactants are required to form AB complex, then it results in higher binding strength and lower value of dissociation constant Kd. The dissociation rate constant of an antibody can be determined by surface plasmon resonance. Generally, surface plasmon resonance analysis measures real-time binding interactions between ligand and analyte (antibodies in solution) by surface plasmon resonance (SPR) using the BIAcore system (Pharmacia Biosensor, Piscataway, N.J.). Surface plasmon analysis can also be performed by immobilizing the analyte and presenting the ligand.

Optional steps can be added to the method of the invention after step b), such as to select the cells producing high affinity antibodies, and/or to isolate the said cells. Thus in a particular embodiment, the method of the invention further comprises
c) selecting cells producing high affinity antigen-specific antibodies; and
d) optionally, before or after the selection step in c) isolating and/or immortalizing said cells.

The term "high affinity antigen-specific antibody" has been already defined in previous paragraphs. In a particular embodiment, the cells selected in step c) are producing high affinity class switched antibodies, preferably selected from the group consisting of IgG and IgA isotypes, more preferably selected from the group consisting of IgG1, IgG2a, IgG3 and IgA isotypes.

Methods for selecting cells producing high affinity antigen-specific antibodies are widely known in the state of the art. Finally, before or after step c), the method of the invention optionally comprises isolating and/or immortalizing said cells.

Examples of this method include, without limiting to, a density gradient such as Ficoll-Hypaque™ (Pharmacia Biotechnology Group, Uppsala, Sweden), magnetic bead assisted separation (MACs and Dynel technologies that are capable of separating the desired components from the rest of the components of the composition.

Methods for isolating cells producing high affinity antigen-specific antibodies include, without being limited to, flow cytometry, cell sorting, or physical dilution. According to the invention, the cells producing high affinity antigen-specific antibodies are B-cells whose surface markers have been defined and explained herein in previous paragraphs.

Methods for immortalizing cells producing high affinity antigen-specific antibodies are widely known in the state of the art. The cells producing high affinity antigen-specific antibodies generated by the method of the present invention are suitable for fusion with a myeloma line for the ultimate production of monoclonal antibodies. Specialized myeloma cell lines have been developed from lymphocyte tumors for use in hybridoma-producing fusion procedures. It is preferred that human myeloma cells are used in the fusion procedure. The myeloma cells are introduced into the system with the inclusion of an agent that promotes the formation of the fused myeloma and B-cells, such as polyethylene glycol (PEG) and Dimethyl sulfoxide (DMSO). Alternatively, fusion can be induced by electrofusion or via fusiogenic viruses such as Sendai virus. Thus, in a particular embodiment, the step (b) of the method of the invention comprises fusing the B cell from step (a) with a cancer cell for obtaining an hybridoma producing the high affinity monoclonal antibody.

Methods for generating hybrids of cells producing high affinity antigen-specific antibodies and myeloma cells usually comprise mixing cells producing high affinity antigen-specific antibodies with myeloma cells in a 2:1 proportion (though the proportion may vary from about 20:1 to about 1:1), respectively, in the presence of an agent or agents that promote the fusion of cell membranes. Fusion procedures usually produce viable hybrids at very low frequency and as such, it is essential to have a means of selecting the fused cell hybrids from the remaining unfused cells, particularly the unfused myeloma cells. A means of detecting the desired antibody-producing hybridomas among other resulting fused cell hybrids is also necessary.

Generally, the selection of fused cell hybrids is accomplished by culturing the cells in media that support the growth of hybridomas but prevent the growth of the myeloma cells which normally would go on dividing indefinitely (the cells used in the fusion do not maintain viability in in vitro culture and hence do not pose a problem.) Generally, the myeloma cells used in the fusion lack hypoxanthine phosphoribosyl transferase. These cells are selected against in hypoxanthine/aminopterin/thymidine (HAT) medium, a medium in which the fused cell hybrids survive due to the HPRT-positive genotype of the spleen cells. The use of myeloma cells with different genetic deficiencies (e.g., other enzyme deficiencies, drug sensitivities, etc.) that can be selected against in media supporting the growth of genotypically competent hybrids is also possible. Several weeks are required to selectively culture the fused cell hybrids. Early in this time period, it is necessary to identify those hybrids which produce the desired antibody so that they may be subsequently cloned and propagated. The detection of antibody-producing hybrids can be achieved by any one of several standard assay methods, including enzyme-linked immunoassay and radioimmunoassay techniques which have been described in the literature.

Once the desired fused cell hybrids have been selected and cloned into individual antibody-producing cell lines, each cell line may be propagated *in vitro* in laboratory culture vessels; the culture medium, also containing high concentrations of a single specific monoclonal antibody, can be harvested by decantation, filtration or centrifugation

In the alternative, the cells producing high affinity antigen-specific antibodies that are generated by the method of the present invention can be suspended in EBV infected culture supernatant and incubated.

The method of the present invention can be used for generating high affinity antigen-specific antibodies on demand. Thus, in another aspect, the invention relates to a high affinity class switched antibody obtained by the method of the invention. Hereinafter "antibody of the invention". In a particular embodiment, the high affinity class switched antibodies are characterized by binding to their antigen with a dissociation constant (KD) of 10-5 to 10-12 moles/L or less. These terms has been explained herein in previous paragraphs.

In another aspect, the present invention comprises a composition comprising the antibody of the invention as defined in the present document.

The antibodies generated by this method can be separated by methods known to those skilled in the art including, but not limited to, precipitation by ammonium sulfate or sodium sulfate followed by dialysis against saline, ion exchange chromatography, ELISA, affinity or immunoaffinity chromatography as well as gel filtration and zone electrophoresis. Antibodies specifically reactive with the antigen produced in accordance with the present invention may be used in any known immunoassays which rely on the binding interaction between an antigenic determinant of a protein and the antibodies. Examples of such assays are radioimmunoassays, enzyme immunoassay (e.g., ELISA), immunofluorescence, immunoprecipitation, latex agglutination, hemagglutination, and histochemical tests.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration and are not intended to be limiting of the present invention.

### BRIEF DESCRIPTION OF THE DRAWINGS

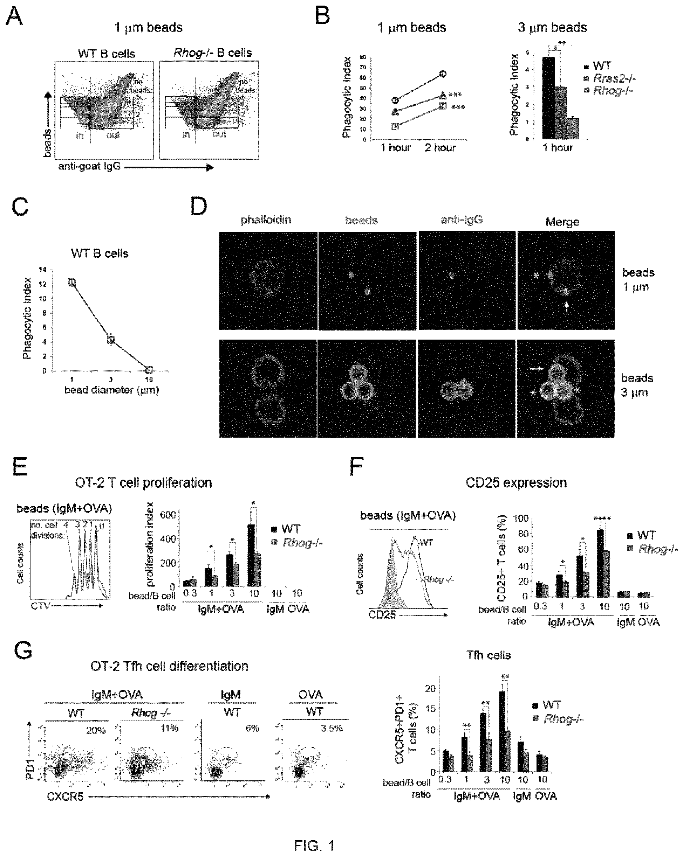

**Figure 1**. B cells can take up antigen and present it to T cells by a phagocytotic mechanism. (A) Flow cytometry plots of WT and RhoG-deficient B cells incubated for 1 h with Crimson fluorescent 1 µm beads coated with goat anti-mouse anti-lgM. Cells were later stained with an anti-goat Alexa 488 antibody to distinguish cells with beads attached to the cell surface (out) from cells having beads exclusively inside (in). Number of phagocytosed beads is calculated by the stepwise increase in Crimson fluorescence intensity. (B) A phagocytic index was calculated according to the number of phagocytic events for B cells incubated for 1 hour or 2 hours with either 1 µm or 3 µm beads coated with anti-lgM. B cells were of wild type (black, circles), Rras2-/- (blue, triangles) or Rhog-/- (red, squares) mice. Data represent the mean ± S.D. (n = 3). * p<0.05; ** p<0.005; *** p<0.0005 (unpaired Student's t test). (C) Phagocytic Index for WT B cells incubated for 1 hour with 1 µm, 3 µm and 10 µm beads coated with anti-lgM. Data represent the mean ± S.D. (n = 3). (D) Optical midplane section of follicular B cells in the process of phagocytosing 1 µm and 3 µm beads coated with anti-lgM. Beads are shown in green, an extracellular staining with anti-goat IgG in red and the cortical actin cytoskeleton in blue (phalloidin staining). Completely phagocytosed beads, negative for anti-goat IgG, are indicated with an arrow, and non-phagocytosed or partly phagocytosed beads are indicated with an asterisk. (E) Proliferation of naive OT-2 CD4+ T cells in response to antigen presentation by B cells preincubated with the indicated bead/cell ratios of 1 µm beads coated with anti-lgM, OVA or anti-lgM plus OVA. A proliferation index was calculated according to the number of cell divisions identified by Cell Trace Violet (CTV) dilution as illustrated in the left panel for T cells incubated with wild type B cells (black) or RhoG-deficient (red). Bar plot to the right represents mean ± S.D. (n = 3). * p<0.05 (unpaired Student's t test). (F) Induction of CD25 expression by OT-2 T cells incubated with phagocytic B cells as in (E) for 3 days. Histogram to the left shows an overlay of CD25 expression in OT-2 cells incubated with: wild type B cells preincubated with beads coated with anti-lgM plus OVA (black line), with RhoG-deficient B cells preincubated with IgM plus OVA (red line) B cells preincubated with beads coated with IgM plus OVA, or with wild type B cells preincubated with beads coated with IgM alone (grey shaded). Bar plot to the right represents mean ± S.D. (n = 3). * p<0.05; ** p<0.005; **** p<0.00005 (unpaired Student's t test). (G) Induction of TFH marker (PD1 and CXCR5) expression in OT-2 T cells after 3 days of culture with WT or RhoG-deficient B as in (E). Bar plots represent the percentage of double positive OT-2 T cells. Data represent the mean ± S.D. (n = 3). ** p<0.005 (unpaired Student's t test).

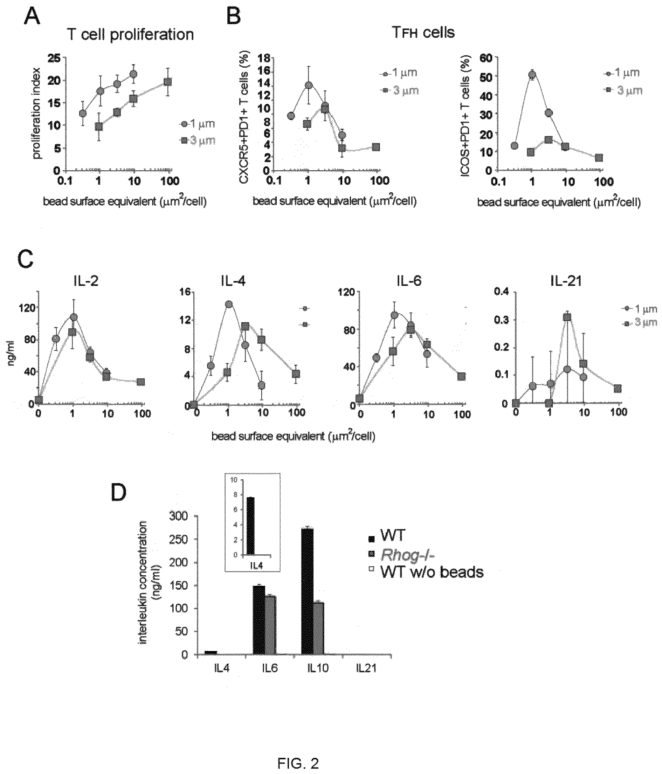

**Figure 2**. Phagocytosis of bead-bound antigen by B cells induces the expression of Tfh markers and release of cytokines by OT2 CD4+ T cells. (A) Proliferation of naive OT2 CD4+ T cells in response to antigen presentation by B cells preincubated with the indicated doses of 1 µm and 3 µm beads coated with NIP-OVA. The dose of beads is normalized according to their exposed surface considering them as spheres. The bead/B cell ratios for 1 µm beads used were 0.1:1 to 3:1; for 3 µm beads were: 0.033:1 to 3.3:1. T cell proliferation was calculated by CTV dilution at day 4 after stimulation. Data represents mean ± S.D. (n = 3). (B) Induction of Tfh marker (PD1 and CXCR5; PD1 and ICOS) expression in OT2 CD4+ T cells after 4 days of culture with WT B cells as in (A). Data represents mean ± S.D. (n = 3).). (C) Cytokine release by OT2 CD4+ T cells incubated with B cells preincubated with the indicated doses of 1 µm and 3 µm beads coated with NIP-OVA as in (A). Cell supernatants were collected at day 4 and cytokine content determined using the multiplexed CBA Array. Data represents mean ± S.D. (n = 3). (D) Cytokine release by OT2 CD4+ T cells incubated with either WT or RhoG-deficient B cells preincubated with a 3:1 1 µm bead/B cell ratio of beads coated with NIP-OVA, or without beads. Cell supernatants were collected at day 7 and cytokine content determined by ELISA. Data represents mean ± S.D. (n = 3).

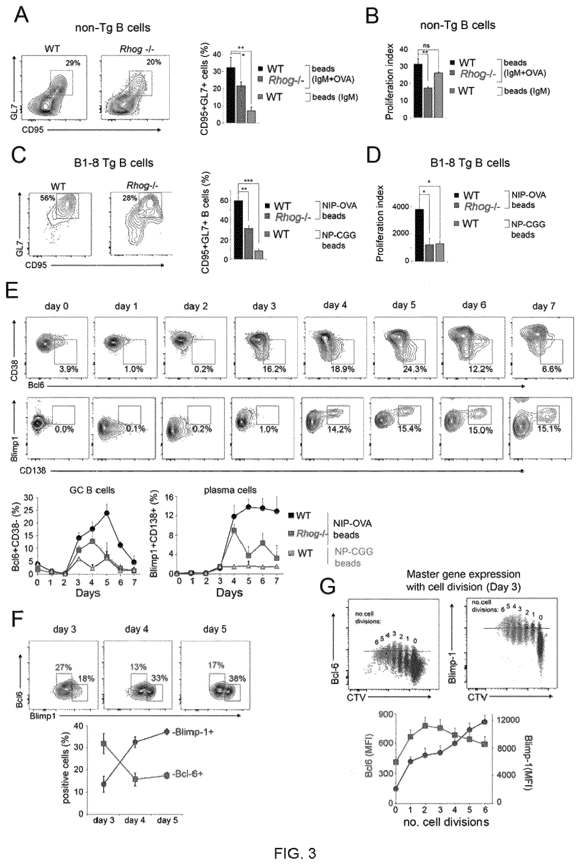

**Figure 3**. B cells differentiate in vitro into GC B cells upon their activation with phagocytic antigen and T-cell help. (A) Naive B cells from WT and Rhog-/- mice were preincubated with 1 µm beads coated with IgM plus OVA or IgM alone, at a 10:1 bead/cell ratio, and co-cultured for 3 days with OT-2 T cells (1:1 B/T cell ratio). FACS contour-plots to the left show the appearance of a double positive (CD95+ GL7+) population in gated B220+ B cells. Bar plot to the right represents mean ± S.D. (n = 3). * p<0.05; ** p<0.005 (unpaired Student's t test). (B) Proliferation of B cells after 3 days of culture was calculated by CTV dilution as in Fig. 1E. Data represent the mean ± S.D. (n = 3). ** p<0.05; ** p<0.005 (unpaired Student's t test). (C) Naive B cells from B1-8hi transgenic WT and Rhog-/- mice were preincubated with 1 µm beads coated with NIP-OVA or NP-CGG, at a 3:1 bead/cell ratio, and co-cultured for 4 days with OT-2 T cells (1:1 B/T cell ratio). FACS contour-plots to the left show the appearance of a double positive (CD95+ GL7+) population in gated B220+ B cells. Bar plot to the right represents mean ± S.D. (n = 3). ** p<0.005; *** p<0.0005 (unpaired Student's t test). (D) Proliferation of B cells from B1-8hi transgenic WT and Rhog-/- mice was calculated after 4 days of culture by CTV dilution as in Fig. 1E. Data represent the mean ± S.D. (n = 3). * p<0.05 (unpaired Student's t test). (E) Differentiation of naive B cells from B1-8hi transgenic WT mice to GC B cells was followed along 7 days of culture in vitro with beads coated with either NIP-OVA or NP-CGG and OT-2 T CD4+ T cells. The percentage of GC B cells was calculated according to GL7 expression and intracellular Bcl-6 expression. Line plot to the right represents mean ± S.D. (n = 3). (F) Expression of master gene regulators of GC (Bcl-6) and plasmacytic cell differentiation (Blimp-1) in function of the number of cell divisions by naive B cells from B1-8hi transgenic WT mice stimulated 3-4 days in vitro with 1 µm beads coated with either NIP-OVA or NP-CGG and OT-2 T CD4+ T cells. Number of cell divisions was assessed by CTV dilution. (G) Expression of Bcl-6 and Blimp-1 measured simultaneously in function of the number of cell divisions by naive B cells from B1-8hi transgenic WT mice stimulated 34 days in vitro with 1 µm beads coated with NIP-OVA and OT-2 T CD4+ T cells. Number of cell divisions was assessed by CTV dilution. Data represent mean ± S.D. (n = 3).

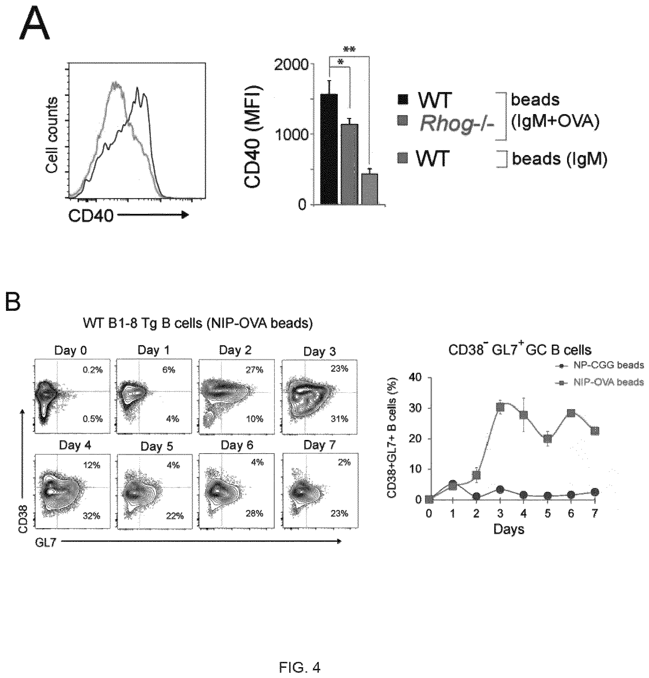

**Figure 4****.** Phagocytosis of bead-bound antigen by B cells induces the expression of GC B cell markers. (A) Non-transgenic B cells from WT and Rhog-/- mice were preincubated with 1 µm beads coated with IgM+OVA or IgM alone, at a 20:1 bead/cell ratio, and co-cultured for 3 days with OT2 T cells (1:1 B/T cell ratio). Overlay histogram to the left show the upregulation of CD40 expression in gated WT B220+ B cells compared to their RhoG-deficient counterparts. Bar plot to the right represents mean ± S.D. (n = 3): * p<0.05; ** p<0.005 (unpaired Student's t test). (B) Differentiation of naive B cells from B1-8hi transgenic WT mice to GC B cells was followed along 7 days of culture in vitro with beads coated with either NP-OVA or NP-CGG and OT2 T CD4+ T cells. The percentage of GC B cells was calculated according to GL7 and CD38 expression. Line plot to the right represents mean ± S.D. (n = 3).

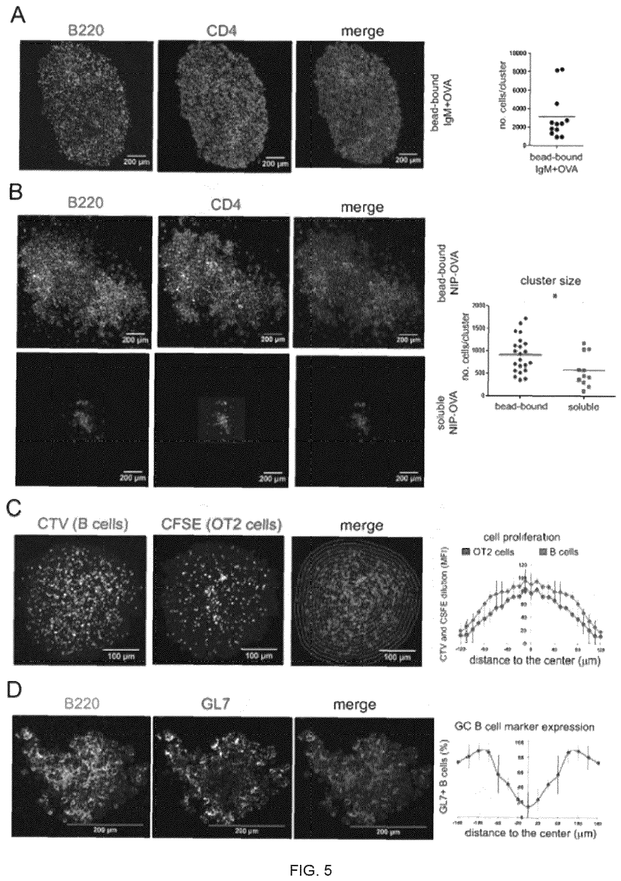

**Figure 5****.** Phagocytic antigen induces the formation of large clusters of intermingled B and T cells. (A) Confocal microscopy image of a large cell cluster generated after 4 days of co-culture of OT-2 T cells and non-transgenic B cells stimulated with 1 µm beads coated with anti-IgM and OVA. B cells are stained with B220 in green; OT-2 T cells with CD4 in red. A quantification of the number of cells per cluster is shown in the plot to the right. (B) Confocal microscopy image of cell clusters generated after 7 days of incubation of naive B cells from B1-8hi transgenic WT mice with OT-2 T cells and either 1 µm beads coated with NIP-OVA or with soluble NIP-OVA. B220 is in green, CD4 in red. Cell number quantification per cluster for both antigen conditions is represented in the graph to the right. Data represent the mean ± S.D. * p<0.05 (unpaired Student's t test). (C) Confocal microscopy image of a cluster of B1-8hi transgenic WT B cells labeled with CTV and cultured for 4 days with CFSE-labeled OT-2 T cells and 1 µm beads coated with NIP-OVA. The intensity of CFSE and CTV staining was measured for all cells placed within the drawn concentric areas and represented in the plot to the right in function of the distance to the center of the cluster. Data represent the mean ± S.D. for n = 5 clusters of similar size. (D) Confocal microscopy of a cluster generated by stimulation of B1-8hi WT B cells with 1 µm beads coated with NIP-OVA for 7 days and stained with the B220 B cell marker (green) and the GL7 GC marker (red). GL7 intensity in function of the distance to the center of the cluster was measured as in Fig. 3C. Line plot to the right represents the mean ± S.D. for n = 5 clusters of similar size.

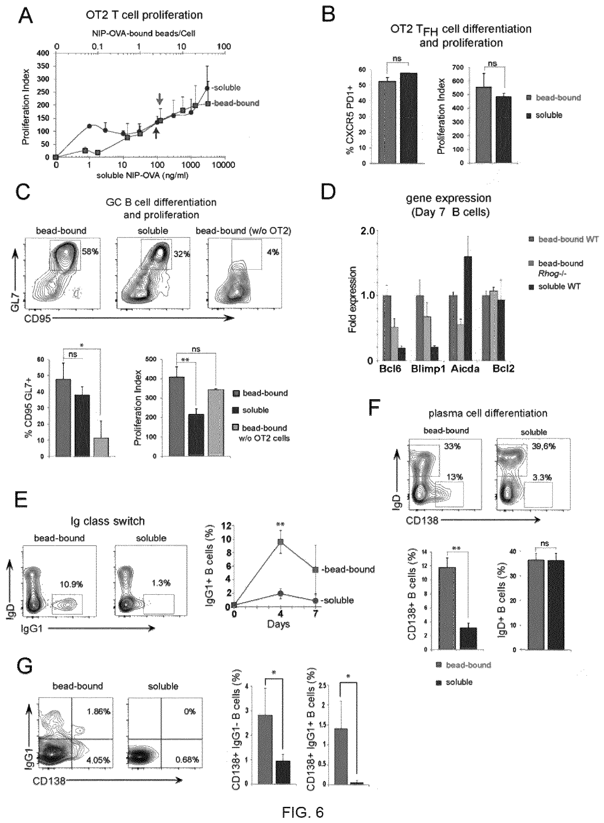

**Figure 6****.** A phagocytic antigen is more efficient than a soluble one at inducing GC B cells in vitro. (A) Proliferation of OT-2 T cells after 4 days of culture with WT B1-8hi B cells stimulated at different doses of bead-bound (red) or soluble (blue) NIP-OVA antigen. Arrows indicate the bead/B cell ratio (3:1) and soluble antigen concentration (100 ng/mL) that induce comparable OT-2 T cell proliferation. (B) Graph plots of TFH markers (CXCR5 and PD1) and proliferation of OT-2 T cells after 4 days of culture with the antigen conditions selected in (A). (C) Contour plots of germinal center marker (CD95 and GL7) expression in B1-8hi B cells after 4 days of culture as in (A). Bar plots below show the percentage of CD95+ GL7+ B cells and B cell proliferation. B1-8hi B cells stimulated with bead-bound NIP-OVA but without OT-2 T cells were used as control (grey). Data represent the mean ± S.D. (n = 3). * p<0.05; ** p<0.005 (unpaired Student's t test). (D) RT-qPCR analysis of expression of the indicated genes performed on sorted WT or Rhog-/- B1-8hi B cells after 7 days of culture with OT-2 and specific stimulus as in (A) and (C). Bar plots show the fold induction expression of genes relative to the bead-bound WT condition. HPRT and 18S were used as normalizers. Data represent the mean ± S.D. (n = 3). (E) Contour plots showing the appearance of Ig class-switched IgG1+ IgD- B1-8hi B cells after 4 days in culture as in (A). Line plot to the right shows the appearance of IgG1+ B cells along 4 and 7 days in culture. Data represent the mean ± S.D. (n = 3). ** p<0.005 (unpaired Student's t test). (F) Contour plots showing the appearance of plasma cells (B220+CD138+ IgD-) after 4 days in culture as in (A). Bar plots below show the percentage of plasma cells (B220int CD138+ IgD-) and non-germinal center (B220hi CD138- IgD+) B cells in those cultures. Data represent the mean ± S.D. (n = 3). ** p<0.005 (unpaired Student's t test). (G) Contour plots illustrates the generation of IgG1+ plasmablasts (B220int IgG1+ CD138+) after 4 days in culture as in (A). Bar plots to the right represent the mean ± S.D. (n = 3). * p<0.05 (unpaired Student's t test).

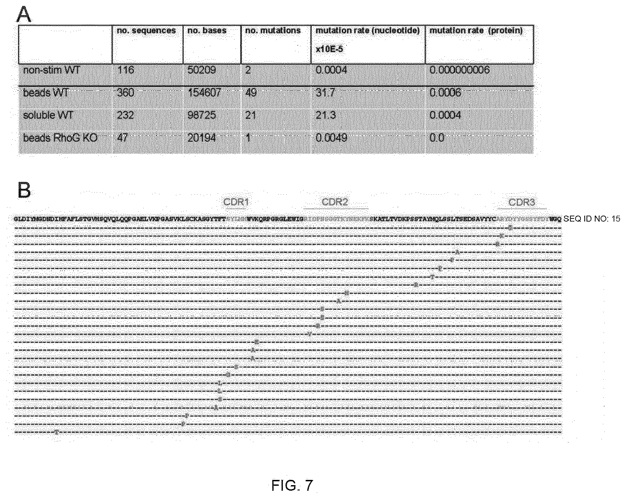

**Figure 7****.** Generation of somatic mutations in IgH V genes in conditions of GC formation in vitro. (A) Number of IgH nucleotide mutations and frequency of non-silent mutations in the IgH V sequence of sorted B cells from B1-8hi transgenic WT or RhoG-deficient mice stimulated with a 3:1 bead/cell ratio of 1 µm beads coated with NP-OVA or with 100 ng/mL of soluble NP-OVA and co-cultured for 7 days with OT2 T cells. (B) Sequences (SEQ ID NO: 15) of the B1-8Vh genes with amino acid replacement mutations detected in the NIP-OVA bead stimulated cultures and their distribution according the 3 CDR regions.

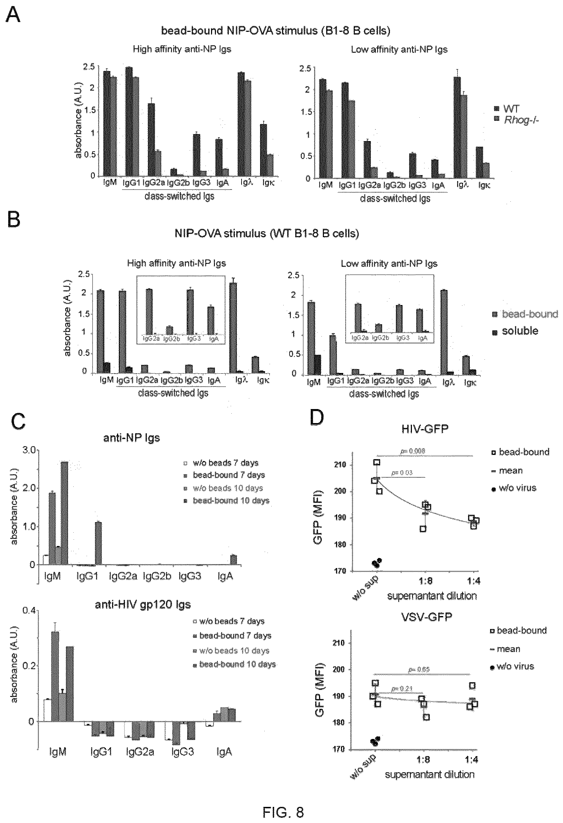

**Figure 8****.** A phagocytic antigen induces the production of high-affinity class-switched and neutralizing antibodies in vitro. (A) Detection of high-affinity and low-affinity anti-NP Igs in supernatants from WT (black) or Rhog-/- B1-8hi B cells cultured for 7 days with OT-2 T cells and bead-bound NIP-OVA (3:1 1 µm bead/B cell ratio). Data represent the mean ± S.D. (n = 3). (B) Detection of high- and low-afinity anti-NP Igs in supernatants of B1-8hi B cells stimulated with soluble (blue) or bead-bound (red) NIP-OVA together with OT-2 T cells for 7 days. Data represent the mean ± S.D. (n = 3). (C) Detection of high affinity anti-NP Igs and anti-HIV Env protein Igs in culture supernatants of non-transgenic B cells stimulated with 1 µm beads (3:1 ratio) coated with NIP-OVA and HIV Env recombinant protein together with OT-2 T cells for 7 days or 10 days. 10-day cell cultures were supplemented at day 5 with 1 ng/ml IL-4 and 10 ng/mL IL-21. Data represent the mean ± S.D. (n = 3). (D) Presence of HIV neutralizing antibodies in the culture supernatants of (C) manifested as the inhibition of entry of GFP-expressing HIV virions coated with either the HIV Env protein or pseudotyped with the VSV G protein in MOLT-4 T cells. Data represent the mean ± S.D. (n = 3). p values were assessed using an unpaired Student's t test.

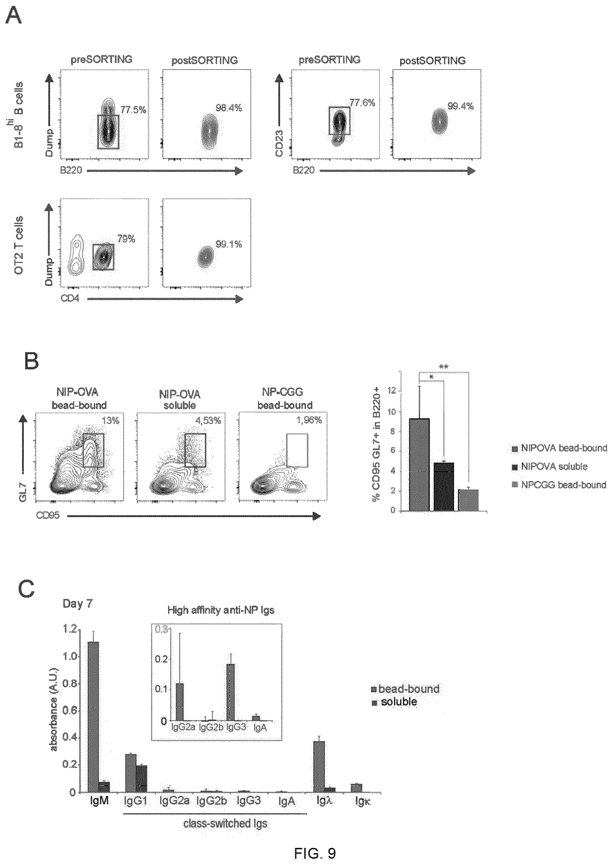

**Figure 9****.** Generation of a GC reaction by phagocytic B cells and helper T cells does not require a third cell type. (A) Sorting of naive follicular B cells from B1-8hi transgenic WT mice and of naive CD4+ T cells from OT2 transgenic WT mice. The Dump channel contain the CD11b+ and CD43+ cells (for follicular B cell sorting) and the B220+, CD11b+, CD8+, NK1.1+, F480+ cells (for CD4+ T cells). (B) Sorted B and T cells as in (A) were incubated with either 1 µm beads coated with NIP-OVA or NP-CGG (3:1 bead:B cell ratio), or with 100 ng/mL soluble NP-OVA for 4 days at a 1:1 B/T cell ratio. FACS contour-plots to the left show the appearance of a double positive (CD95+ GL7+) population in gated B220+ B cells. Bar plot to the right represents mean ± S.D. (n = 3): * p<0.05; ** p<0.005 (unpaired Student's t test). (C) Detection of high-affinity anti-NP Igs in supernatants from B and T cell cultures prepared as in (A) and (B) incubated for 7 days (3:1 1 µm bead/B cell ratio) and soluble antigen concentration. Data represent the mean ± S.D. (n = 3).

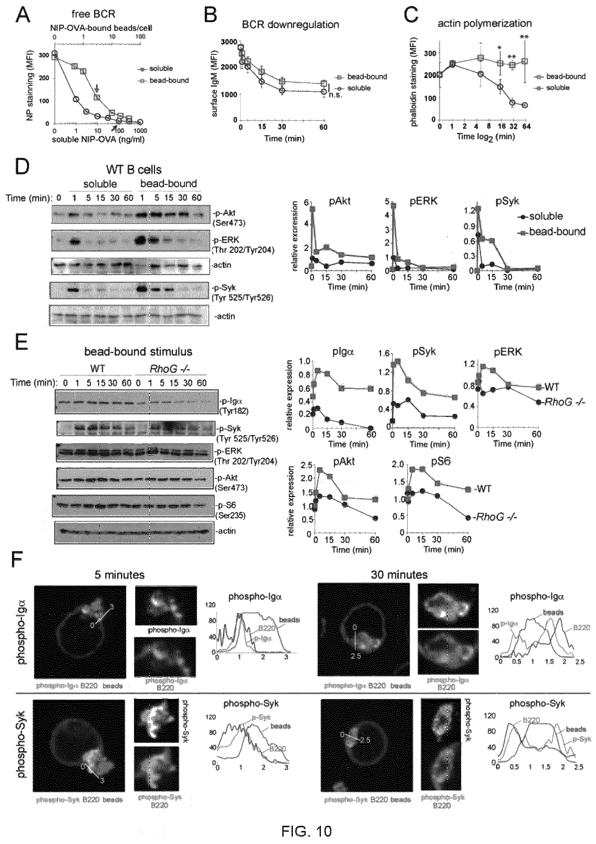

**Figure 10****.** A phagocytic antigen induces a stronger and more sustained BCR signal than a soluble one. (A) Surface BCR saturation plot of purified B1-8hi B cells incubated with bead-bound (red) or soluble NIP-OVA (blue) antigen at different doses for 1 hour at 0°C. Free unbound BCR was estimated by staining with NP-PE. Data represent the mean ± S.D. (n = 3). Arrows indicate the bead-dose and soluble concentration determined previously with comparable effects on OT-2 T cell proliferation (Fig. 4A). (B) BCR downmodulation was estimated according to anti-IgM staining of B1-8hi B cells after stimulation with bead-bound (red, 3:1 ratio) or soluble (blue, 100 ng/ml) NIP-OVA antigen for different time-points at 37°C. Data represent the mean ± S.D. (n = 3). (C) F-actin content was measured by phalloidin staining of B1-8hi B cells after stimulation with bead-bound (red, 3:1 ratio) or soluble (blue, 100 ng/ml) NIP-OVA antigen for different time-points at 37°C. Data represent the mean ± S.D. (n = 3). * p<0.05; ** p<0.005 (unpaired Student's t test). (D) Immunoblot analysis of phosphorylation events downstream of the BCR after stimulation of WT B1-8hi B cells with either a 3:1 ratio of bead-bound NIP-OVA or with 100ng/ml of soluble NIP-OVA for different time-points. Plots to the right show protein phosphorylation levels relative to the amount of actin quantified by densitometry. (E) Immunoblot analysis of phosphorylation events downstream of the BCR after stimulation of WT (red) or RhoG-deficient (blue) B1-8hi B cells with a 3:1 ratio of bead-bound NIP-OVA for different time-points. Plots to the right show protein phosphorylation levels relative to the amount of actin quantified by densitometry. (F) Midplane confocal microscopy images of B1-8hi B cells in the process of phagocytosing (5 min. of incubation) or having completely phagocytosed (30 min.) 1 □m beads coated with NIP-OVA. Details of the phagocytic cups (5 min) and the phagosomes (30 min.) are shown in the enlarged pictures. The B220 B cell marker is in blue, beads in red and phospho-Ig□ and phospho-Syk antibodies in green. Histogram overlays show the signal intensity in the 3 colors along the white lines drawn in the main images.

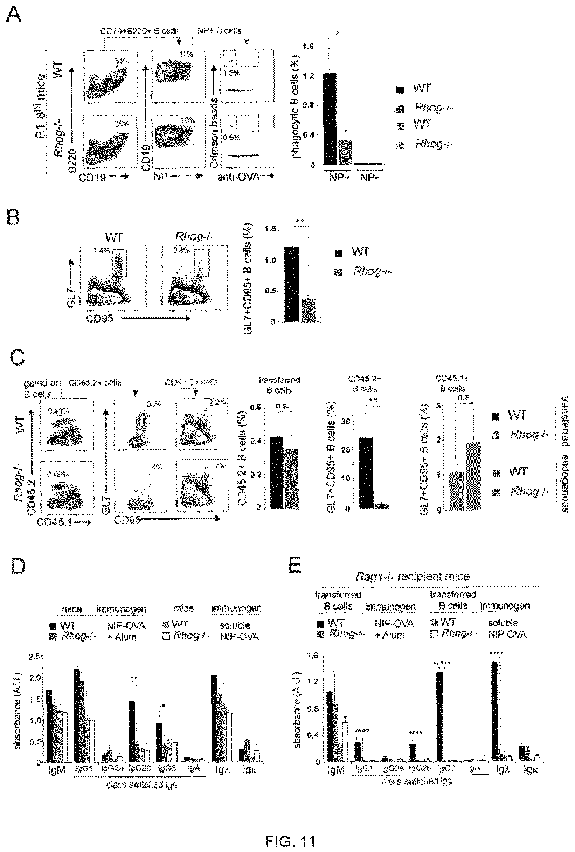

**Figure 11****.** Phagocytosis of antigen by B cells mediates the adjuvant effect of alum in vivo. (A) Phagocytosis of 1 µm fluorescent (Crimson) beads covalently bound to NIP-OVA by spleen B cells of WT (black; grey) or Rhog-/- (red; orange) B1-8hi mice was assessed 5 hours after bead administration i.p. Splenic B cells were identified by double labeling with CD19 and B220 and within B cells, the BCR transgene bearing population by staining with NP-PE. The percentage of B cells that had phagocytosed beads was calculated according to the acquisition of Crimson dye fluorescence and negative staining with extracellular anti-OVA. Plot to the right represents the mean ± S.D. (n = 3). * p<0.05 (unpaired Student's t test). (B) Expression of GC GL7 and CD95 B cell markers in WT (black) and Rhog-/- (red) mice 7 days after i.p immunization with NIP-OVA covalently bound to 1 µm beads. Plot represents the mean ± S.D. (n = 3) of selected populations. ** p<0.005 (unpaired Student's t test). (C) Adoptive transfer of purified CD45.2+ B cells from WT (black) or Rhog-/- (red) mice to WT CD45.1+ recipient mice was followed by i.p immunization with NIP-OVA covalently bound to 1 µm beads. Seven days later spleen cells were analyzed for CD95 and GL7 GC marker expression within the transferred CD45.2+B220+ and the endogenous CD45.1+B220+ populations. Plots represent the mean ± S.D. (n = 3) of selected populations. ** p<0.005; n.s., not significant (unpaired Student's t test). (D) Detection of high-affinity anti-NP Igs in sera from WT (black; grey) or Rhog-/- (red; white) non-transgenic mice 20 days after the first immunization either with soluble NIP-OVA or a NIP-OVA plus alum complex. Data represent the mean ± S.D. (n = 3). ** p<0.005 (unpaired Student's t test). (E) Detection of high- and low-affinity anti-NP Igs in sera from Rag1-/- mice adoptively transferred with purified OT-2 CD4+ T cells and either WT (black; grey) or Rhog-/- (red; white) B cells and immunized with NIP-OVA plus alum complex. Sera were taken 16 days after the first immunization. Data represent the mean ± S.D. (n = 2). **** p<0.00005; ***** p<0.000005 (unpaired Student's t test).

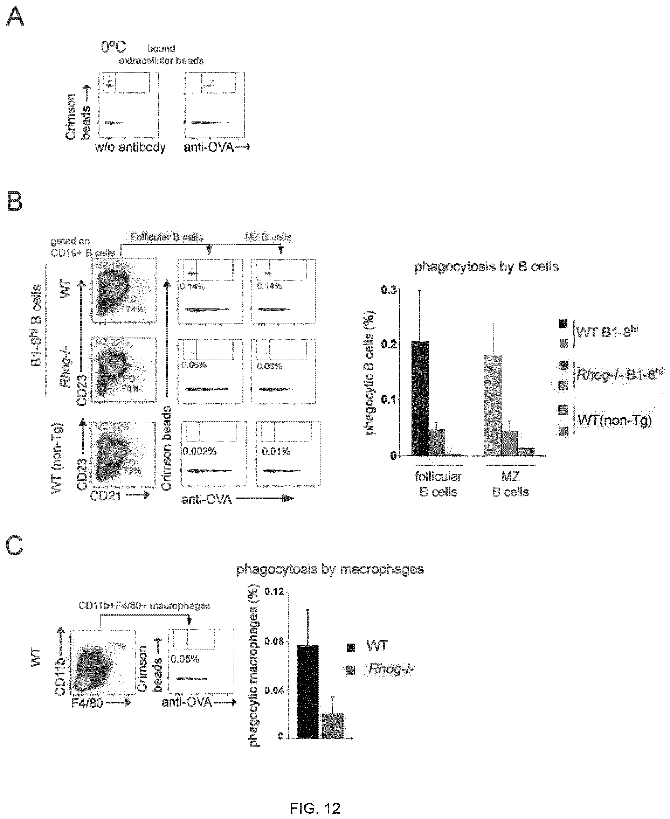

**Figure 12****.** Splenic follicular and MZ B cells phagocytose antigen-coated beads by a RhoG-dependent process. (A) WT spleen B1-8hi B cells were incubated in vitro with fluorescent 1 mm beads coated with NIP-OVA at 0°C for 1 hour and subsequently were stained or not with an anti-OVA antibody. B cells that had bound beads but did not internalize them were Crimson+ and positive for anti-OVA staining. (B) Phagocytosis of 1 µm fluorescent (Crimson) beads covalently bound to NIP-OVA by spleen B cells of WT (black; grey) or Rhog-/- (red; orange) B1-8hi mice was assessed 5 hours after bead administration i.p. Follicular (FO) and marginal zone (MZ) splenic B cells were identified within the CD19+ population by CD21 and CD23 staining. Non-transgenic WT mice was inoculated in parallel as a control group. The percentage of follicular and MZ B cells that had phagocytosed beads was calculated according to their positivity to Crimson dye and negative staining with extracellular anti-OVA. Plot to the right represents the mean ± S.D. (n = 3). * p<0.05 (unpaired Student's t test). (C) Phagocytosis of beads inoculated i.p. by spleen macrophages was assessed in the samples studied in B after staining with CD11b and F4/80 markers. Plot to the right represents the mean ± S.D. (n = 3). * p<0.05 (unpaired Student's t test).

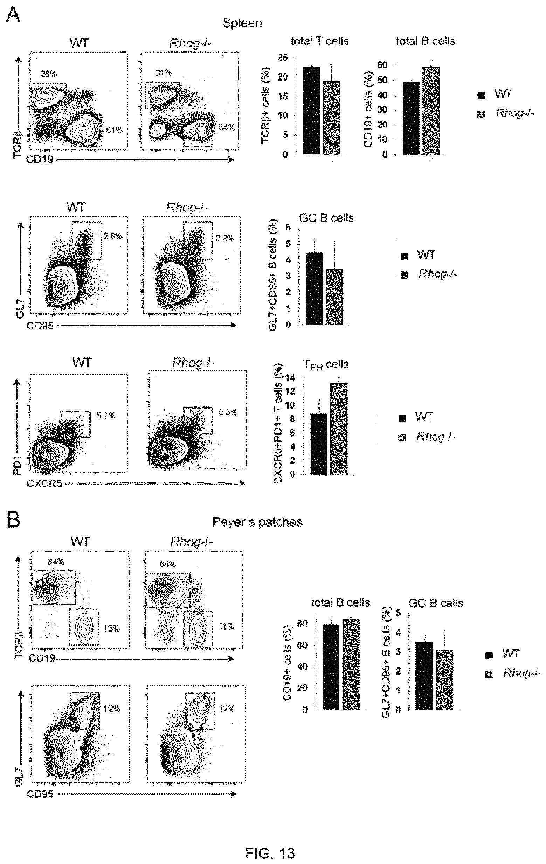

**Figure 13****.** Rhog-/- mice are not deficient in the GC response to immunization with SRBC. (A) Percentage of T and B cells and of Tfh and GC B cells in the spleen of WT and Rhog-/- mice 7 days after immunization with SRBC. (B) Percentage of T and B cells and of GC B cells in Peyer's Patches of non-immunized WT and Rhog-/- mice. Bar plots represents mean ± S.D. (n = 3 mice per group).

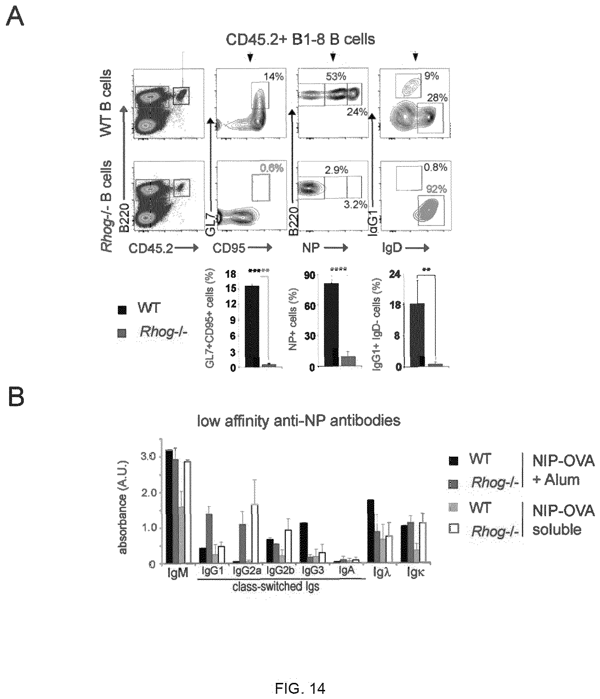

**Figure 14****..** Phagocytosis of antigen by B cells mediates the adjuvant effect of alum in vivo. (A) Adoptive transfer of purified CD45.2+ B cells from WT (black) or Rhog-/- (red) mice to WT CD45.1 + recipient mice was followed by i.p immunization with NIP-OVA plus alum complex. Seven days later spleen cells were analyzed for CD95 and GL7 GC marker expression, NP-binding capability and IgG1 expression within the transferred CD45.2+B220+ population. Plots represent the mean ± S.D. (n = 3) of selected populations. ** p<0.005 **** p<0.00005; ***** p<0.000005 (unpaired Student's t test). (C) Detection of low-affinity anti-NP Igs in sera from Rag1-/- mice adoptively transferred with purified OT-2 CD4+ T cells and either WT (black; grey) or Rhog-/- (red; white) B cells and immunized with NIP-OVA plus alum complex. Sera were taken 14 days after immunization. Data represent the mean ± S.D.

### EXAMPLE

### I. MATERIALS AND METHODS

### Mice

Rras2-/- and Rhog-/- mice were generated as previously described (Delgado, et al. (2009). Nat Immunol. 10, 880-888; Vigorito, E. et al. (2004). Mol Cell Biol. 24, 719-729). Those mice were crossed with NP-specific B1-8hi knocking mice bearing a pre-rearranged V region (Shih, T.A., et al. (2002). Nat Immunol. 3, 399-406). Mice transgenic for the OT-2 TCR specific for a peptide 323-339 of chicken ovoalbumin presented by I-Ab (Barnden, M.J., et al. (1998). Immunol Cell Biol. 76, 34-40) and C57BL/6 bearing the pan-leukocyte marker allele CD45.1 were kindly provided by Dr. Carlos Ardavín (CNB, Madrid). Mice carrying the Rag1tm1Mom mutation in homozygosis lack both T and B cells (Mombaerts, P., et al. (1992). Cell. 68, 869-877) and were kindly provided by Dr. Cesar Cobaleda (CBMSO, Madrid). All animals were backcrossed to the C57BL/6 background for at least 10 generations. For all in vivo experiments age (6-10 weeks) and sex were matched between the Rhog+/+ (WT) and Rhog-/- mice. Mice were maintained under SPF conditions in the animal facility of the Centro de Biología Molecular Severo Ochoa in accordance with applicable national and European guidelines. All animal procedures were approved by the ethical committee of the Centro de Biología Molecular Severo Ochoa.

### Antigen-coated bead preparation

To prepare beads with adsorbed antigen, carboxylated latex beads of 1 µm diameter, a total of 130x10⁶ beads were incubated overnight with a concentration of 40 µg/ml of protein in 1 mL of PBS at 4°C. For preparation of antigen-coated beads of 3 and 10 µm diameter the concentration of beads was reduced in staggered way; 3-fold and 30-fold, respectively. Beads were subsequently washed twice with PBS plus 1% BSA and resuspended in RPMI medium. To prepare beads with covalently bound antigen, the PolyLink Protein Coupling Kit (Polysciences) was used as indicated by the manufacturer. An equivalent to 12.5 mg of beads were washed in Coupling Buffer (50 mM MES, pH 5.2, 0.05% Proclin 300), centrifuged 10 minutes at 1000g and resuspended in 170 µL Coupling Buffer. A 20 µL volume of Carbodiimide solution (freshly prepared at 200 mg/mL) was added to the bead suspension and incubated for 15 minutes. After that, a total of 400 µg of NIP-OVA were added at a concentration of 5 mg/ml final concentration. To prepare beads coupled to two different proteins we followed a sequential procedure: the first protein was added at sub-saturating conditions (100 µg p17/p24/gp120 HIV-1 protein) for one hour and after that the second one was added to reach saturation (400 µg NIP-OVA) and incubated one additional hour. Incubations were carried out at room temperature with gentle mixing. Beads were centrifuged and washed twice in Wash/Storage buffer (10 mM Tris, pH 8.0, 0.05% BSA, 0.05% Proclin 300). To remove noncovalent bound protein, beads were washed once with 0.1% SDS followed by two washes with PBS + 1% BSA to remove the SDS.

### Phagocytosis assays

Naïve B2 cells were resuspended in RPMI containing 20 mM Hepes plus 0.2% BSA and plated in 96 well V-bottom plates at a concentration of 1x10⁶ cells in 50 µl. Antibody-coated beads were added to reach a bead:cell ratio of 10. The cell and bead suspension was briefly centrifuged at 1,500 rpm and was incubated at 37°C for different time points. Subsequently, cells were washed and stained on ice with a fluorescent isotype-specific Ig antibody to track the presence of beads bound to the cells that had not been phagocytosed. At this stage, the cells were either analysed by flow cytometry (FACS Canto II) or incubated for 15 minutes on coverslips coated with poly-L-lysine and then processed for immunofluorescence.

### Proliferation and stimulation assays

Proliferation of OT-II and B cells was assessed using CFSE or Cell Tracer Violet (CTV) labelling as specified by the manufacturer (Thermofisher). A total of 2x10⁵ purified naive B cells were CTV-stained and co-cultured with purified CFSE-stained OT-II T cells at a 1:1 ratio together with antigen coated-beads or soluble antigen in a round-bottom 96 well plate. For the bead-bound stimulus, B cells were incubated with 1 µm beads coated with NIP-OVA, NP-CGG or anti-IgM plus ovalbumin at different bead:B cell ratios. For stimulation with soluble antigen, NIP-OVA was used at a concentration of 100 ng/mL. After 3-4 days of culture, cells were washed in PBS plus 1% BSA and stained for T cell activation (CD25, CD44), Tfh (CXCR5, PD1, ICOS) or germinal center B cell (CD95, GL7, CD38) markers. To study differentiation of these cultured B cells to plasma cells, the cells were left on culture for 4 and 7 days and stained for CD138, IgD, and IgG1. The intracellular staining for Bcl-6 and Blimp1 were performed using the Foxp3/ Transcription Factor Staining Buffer Set. Samples were analysed by FACS (FACS Canto II) and FlowJo software.

### Measurement of antigen-specific antibodies

To measure the release of NP-specific Ig *in vitro* B cell: OT-2 T cell culture supernatants were incubated on NP(7)-BSA-coated or NP(41)-BSA coated Costar p96 flat-bottom plates to measure the release of high- and low-affinity Igs, respectively. The SBA Clonotyping System-HRP (Southern Biotech) was used to detect the presence of antigen-specific Ig isotypes. When B1-8hi transgenic B cells were used, B cells and OT-2 T cells were cultured at 1:1 ratio for 4 or 7 days in the presence of NIP-OVA-coated 1 µm beads (3:1 bead/B cell ratio) or 100ng/mL soluble NIP-OVA. For cultures of non-transgenic B cells, purified naive C57BL/6 B2 cells were preincubated with a mixture of NIP-OVA and HIV-1 p17/p24/gp120 fusion protein (Jena Biosciences) covalently bound to 1 µm beads (1 bead:Bcell ratio) and cultured with OT-2 T cells (1:1 B cell/OT-2 T cell ratio). After 5 days of culture, some cultures were supplemented with 1 ng/mL IL-4 and 10 ng/mL IL-21 (Prepotech). Supernatants were recovered at day 10 and used to measure Igs by ELISA.

In immunized mice, sera were obtained after 7 days of first immunization with 200 µg of NIP-OVA embedded with Alum or PBS, or 10x106 1µm beads covalently bound to NIP-OVA. After 14 days from first immunization, mice were reimmunized and sera were obtained one week later. NP(7)-BSA and NP(41)-BSA plate bound were also used to measure high and low-affinity immunoglobulins. SBA Clonotyping System-HRP (Southern Biotech) was used to perform the ELISA.

### HIV neutralization assay

Lentiviral supernatants were produced from transfected HEK-293T cells as described previously (Martinez-Martin, N., et al. (2009). Sci Signal. 2, ra43.). Briefly, lentivirus were obtained by co-transfecting plasmids psPAX2 (gag/pol), pHRSIN-GFP (Provided by J.A. Pintor) and either HIV-1 envelope (pCMV-NL4.3) or VSV envelope (pMD2.G) using the JetPEI transfection reagent (Polyplus Transfection). Viral supernatant were obtained after 24 and 48 hours of transfection. Polybrene (8 µg/mL) was added to the viral supernatants previous transduction of MOLT-4 cells (ATCC® CRL-1582™). A total of 3x10⁵ MOLT-4 cells were plated in a p-24 flat-bottom well and 700 µL of viral supernatant were added to them. Cells were centrifuged for 90 minutes at 2,200 rpm and left in culture for 24 hours.

The culture supernatants of purified naive C57BL/6 B2 cells stimulated with a mixture of NIP-OVA and HIV-1 p17/p24/gp120 fusion protein covalently bound to 1 µm beads (1 bead:Bcell ratio) together with OT-2 T cells (1:1 B cell/OT-2 T cell ratio) were incubated at different dilutions (1:8 and 1:4) with the viral supernatant for 1 hour at 37 °C and subsequently the mixture was used to infect MOLT-4 cells. As a control of infectivity, MOLT-4 cells were infected with viral supernatant without antibody supernatants. MOLT-4 cell infection was assessed according to GFP expression by Flow Cytometry (FACS Canto II).

### In vivo phagocytosis assay

B1-8hi mice were immunized intraperitoneally with 2x10⁷ Crimson fluorescent beads of 1µm covalently bound to NIP-OVA. Spleens were harvested after 5 hours and were disintegrated in PBS+2% FBS on ice. Cell suspensions were stained with antibodies to identify macrophagues, (CD11b and F4/80), B cells (CD19 and B220), marginal zone and follicular B cells (CD23 and CD21). To identify those beads phagocytosed from those just attached to the membrane, cells were stained with anti-Ovalbumin-FITC 1:100 dilution for 30 minutes. Samples were analysed by Flow Cytometry (FACS Canto II). All ex-vivo procedures were performed at 0°C.

### Adoptive transfer and immunizations

To assess the formation of GC B cells in vivo, 6- to 12-week-old mice were immunized intraperitoneally (i.p.) with 2 x 10⁹ SRBC as described previously (Aiba, Y.,et al. (2010). Proc Natl Acad Sci USA. 107, 12192-12197). Spleens were harvested 7 days post-injection (p.i.). To assess the formation of GC B cells in vivo and the generation of anti-NP antibodies, mice were immunized i.p. with 200 µg of soluble NIP-OVA in 200 µl of PBS. Alternatively, mice were immunized with 200 µg of NIP-OVA complexes with 100 µl of Alum diluted 1:1 with PBS. For immunization with NIP-OVA bound to beads, a total of 20x106 1 µm beads covalently bound to NIP-OVA were administered i.p. in 200 µL PBS.

For adoptive transfer into CD45.1 mice, 1x10⁷ purified B cells from spleens of B1-8 WT and RhoG-/- were injected intravenously. Acceptor mice were immunized with 200 µgr NIP-OVA embedded with Alum intraperitoneally or with 2x10⁷ of 1 µm beads bound covalently to NIP-OVA.
For adoptive transfer into Rag1-/- mice, 1x10⁵ purified CD4 T cells from lymph nodes and spleen of OT-2 mice and 1x10⁶ purified B cells from spleen of B1-8hi WT or Rhog-/- mice were injected intravenously. Acceptor mice were immunized intraperitoneally with 200 µg NIP-OVA either mixed with Alum or in PBS. After 14 days from first immunization, acceptor mice were reimmunized in the same conditions.

Soluble immunoglobulins were quantified by ELISA for isotype determination (Southern Biotech) with a 1:100 dilution of the sera from the immunized mice.

### Cell preparation and purification

The lymph nodes and spleen from 6-8 weeks mice were homogenized with 40 µm strainers and washed in phosphate-buffered saline (PBS) containing 2% (vol/vol) fetal bovine serum (FBS). Spleen cells were resuspended for 3 minutes in AcK buffer (0.15 M NH4CI, 10mM KHCO3, 0.1 mM EDTA, pH 7.2-7.4) lo lyse the erythrocytes and washed in PBS 2% FBS. B cells from spleen were negatively selected using a combination of biotinylated anti-CD43 and anti-CD11b antibodies and incubation with streptavidin beads (Dynabeads Invitrogen) for 30 minutes and separated using Dynal Invitrogen Beads Separator. B1-8hi B cells were purified using biotinylated anti-CD43, anti-CD11b and anti-kappa antibodies. OT-2 T cells from lymph nodes and spleen were purified using a mix of biotinylated antibodies: anti-B220, anti-CD8, anti-NK1.1, anti-CD11b, anti-GR1, and anti-F4/80. Splenic and lymph node B and T cells were maintained in RPMI 10% FBS supplemented with 2 mM L-glutamine, 100U/mL penicillin, 100 U/mL streptomycin, 20 µm β-mercaptoethanol and 10 mM sodium pyruvate.

### Real-time PCR

A total of 5x10⁶ purified B1-8hi WT or Rhog-/- B2 cells were cultured with purified OT2 (ratio 1:1) and different BCR stimuli (NIP-OVA bound to 1 µm beads, 100 ng/mL soluble NIP-OVA or NP-CGG bound to 1 µm beads) in a 6 well flat-bottom plate. After 7 days, B cells were sorted (FACSAria Fusion (BSC II)) and their RNA was isolated using the RNAeasy Plus Mini Kit (QIAGEN). cDNA was synthetized with SuperScript III (Invitrogen) using Oligo-dT primers. Quantitative real-time PCR was performed in triplicate using the reverse transcription reaction with SYBR Green PCR Master Mix, gene-specific primers and ABI 7300 Real Time PCR System. Obtained cycle threshold (Ct) values were used to calculate mRNA levels relative to the HPRT and GAPDH expression using the 2-ΔΔCt method.

### BCR downmodulation

Purified B1-8hi B cells were resuspended in RPMI plus 10% FBS at a density of 2.5 x10⁵ cells/well in a p96 V-bottom plate in a total volume of 50 µL. Stimulus (NIP-OVA bound to beads in a 3:1 ratio or soluble NIP-OVA at 100 ng/mL concentration) were added to the wells and incubated at 37°C for different time points. After appropriate incubation time, cells were stained for IgM, CD19 and B220 at 0°C and analysed by FACS.

### Confocal microscopy

For immunofluorescence analysis of B:T cell clusters, purified B cells were cultured with purified OT2 T cells for 4 or 7 days in 6 well flat-bottom plates with either 1µm beads coated with anti-IgM plus ovalbumin or NIP-OVA or 100 ng/mL soluble NIP-OVA. Afterwards, cells were fixed in 4% paraformaldehyde for 20 minutes and transferred to poly-L-lysine treated coverslips. Cells were stained for 1 hour at 0°C with antibodies specific for B220, CD4 and GL7. For analysis of intracellular phosphorylation, purified B1-8hi B cells were starved for one hour in RPMI plus 20mM HEPES-HCI pH=7.4, and subsequently stimulated with NIP-OVA-coated fluorescent-beads (3 bead:B cell ratio) or soluble NIP-OVA (100ng/mL). Cells were fixed in 4% paraformaldehyde at 0°C for 20 minutes to stop the stimulus, washed with Tris-buffered saline (TBS), and adhered to poly-L-lysine treated coverslips. Extracellular staining for B220 was performed in TBS for 1 hour at 0 °C. After that, cells were stained for anti-phospho-Syk and anti-phospho-Igα as suggested by the manufacter (Cell Signaling). Confocal images were acquired with a Zeiss LSM710 system and a Zeiss AxioObserver LSM710 Confocal microscopes.

### Measurement of actin polymerization

Purified B1-8hi B cells were starved as above and stimulated with either NIP-OVA bound to beads or soluble NIP-OVA for different times at 37 °C. After that, cells were washed once in PBS plus 1 % BSA at 37 °C and fixed with 4% paraformaldehyde for 10 minutes at room temperature. Extracellular staining for B220 was performed in PBS plus 1% BSA. After washing, cells were permeabilized in 4% paraformaldehyde plus 0.1% Triton X-100 for two minutes at room temperature. Phalloïdin-Alexa488 was diluted 1:200 in PBS plus 1%BSA and incubated with cells for 1 hour. After washing, cells were analysed by Flow Cytometry (FACS Canto II).

### NP saturation assay

A total of 1x10⁵ purified B1-8hi B cells/well were plated in a V bottom 96 well plates and incubated with different doses of stimuli for one hour at 0°C. Cells were washed once with cold PBS plus 1% BSA and stained with NP(36)-PE at 2.5 µg/ml in 50 µL per well at 0°C for 30 minutes. Cells were washed once with cold PBS plus 1% BSA and analyzed by Flow Cytometry (FACS Canto II).

### Immunoblot analysis of B cell activation

Purified B1-8 B cells were resuspended in RPMI plus 20 mM Hepes and left in starving conditions for 1 hour. Cells were stimulated at different time-points with NIP-OVA bound-beads (ratio 3:1 beads/B cell) or 100 ng/mL soluble NIP-OVA. After stimulation, cells were lysed in Brij96 lysis buffer containing protease and phosphatase inhibitors (1% Brij96, 140 mM NaCl, 10 mM Tris-HCl [pH 7.8], 10 mM iodoacetamide, 1 mM PMSF, 1 µg/mL leupeptin, 1 µg/mL aprotinin, 1 mM sodium orthovanadate, 20 mM sodium fluoride and 5 mM of MgCl2). Immunoblotting was performed as described previously (Martinez-Martin et al., 2009, cited *ad supra*)

### Somatic hypermutation

Purified WT or RhoG-/- B1-8 B cells were cultured with purified OT-II T cells (ratio 1:1) and soluble NIP-OVA (100ng/mL) or bead-bound NIP-OVA (ratio 3:1 beads/Bcell). After 7 days of culture, B1-8 cells were sorted and their genomic DNA was extracted using QIAamp DNA kit (QIAGEN). B1-8Vh genes were amplified by PCR with the Expand High Fidelity (Roche) and the primers forward 5'-CCATGGGATGGAGCTGTATCATCC-3' (SEQ ID NO: 1) and reverse 5'-GAGGAGACTGTGAGAGTGGTGCC-3 (SEQ ID NO: 2) as described previously (Shih, T.A., et al. (2002). Nat Immunol. 3, 399-406). PCR products were subcloned into PCR2.1 vector (Invitrogen). DH5α bacteria were transformed with the subcloned products. Individual DH5α clones grown in LB+Ampicillin were selected for sequencing using the SUPREMERUN 96 system of GATC.

### Interleukin measurement

Purified WT B1-8hi B cells were cultured together with OT2 T cells and two different bead sizes (1 µm and 3 µm) bound to NIP-OVA at different doses. After 4 days, the supernatant was used to measure cytokines with a BD Cytometric Bead Array (CBA) Kit as indicated by the manufacturer. In cultures of WT or RhoG-deficient B1-8hi B cells with purified OT2 T cells and NIP-OVA 1 µm bead-bound, the supernatant was obtained after 7 days.

### Statistical analysis

Statistical parameters including the exact value of n, the means +/- s.d. are reported in the Figure and Figure legends. A non-parametric 2-tailed unpaired t-test was used to assess the confidence intervals.

### Origin of the reagents used in the illustrative example of the invention

**Table 1. Sequence-Based Reagents**

| **Gene** | **Primers** |
|---|---|
| **Bcl6** | FW GGAAGTTCATCAAGGCCAGT (SEQ ID NO: 3) |
| | RV GACCTCGGTAGGCCATGA (SEQ ID NO: 4) |
| **Bcl2** | FW GTACCTGAACCGGCATCTG (SEQ ID NO: 5) |
| | RV GGGGCCATATAGTTCCACAA (SEQ ID NO: 6) |
| **Blimp1** | FW GGCTCCACTACCCTTATCCTG (SEQ ID NO: 7) |
| | RV TTTGGGTTGCTTTCCGTTT (SEQ ID NO: 8) |
| **Irf4** | FW GGAGTTTCCAGACCCTCAGA (SEQ ID NO: 9) |
| | RV CTGGCTAGCAGAGGTTCCAC (SEQ ID NO: 10) |
| **HPRT** | FW TCCTCCTCAGCAAGCTTTT (SEQ ID NO: 11) |
| | RV CCTGGTTCATCATCGCTAATC (SEQ ID NO: 12) |
| **GAPDH** | FW CTCCCACTCTTCCACCTTCG (SEQ ID NO: 13) |
| | RV CATACCAGGAAATGAGCTTGACAA (SEQ ID NO: 14) |

### II. RESULTS

### B2 cells phagocytose and present antigen by a RhoG-dependent mechanism.

To study the RRas2 and RhoG dependence of BCR-triggered B cell phagocytosis, we incubated primary spleen B cells from wild type, Rras2-/- or Rhog-/- mice for 1-2 hours with 1 µm diameter fluorescent latex beads coated with an anti-IgM antibody and determined their internalization (phagocytosis) by flow cytometry. To restrict the assay to B2 B cells, B220+CD43-CD11b- lymphocytes were gated on. In order to distinguish totally phagocytosed beads from beads just adhered to the B cell surface, the cell cultures were incubated with a fluorescent anti-goat IgG antibody able to recognize the anti-IgM antibody used to coat the beads. Cells with beads adhered to their cell membrane could be identified by their positivity to the secondary anti-goat IgG antibody and the number of bound beads was calculated by the stepwise increase in bead fluorescence intensity (Fig. 1A). Cells positive for bead fluorescence and negative for the anti-goat IgG antibody were considered as cells that had totally phagocytosed the beads, which would make them inaccessible to the antibody. The stepwise increase in fluorescence intensity allowed calculation of a parameter -phagocytic index- that reflected the number of phagocytic events. B cells deficient in either RRas2 or RhoG were also deficient in the phagocytosis of 1 µm beads (Fig. 1B, left). The effect of RRas2 and RhoG deficiencies was even more prominent when the bead diameter was increased to 3 µm, suggesting that the requirement for these GTPases is more stringent for bigger particles (Fig.1B, right). Indeed, phagocytosis of 10 µm beads was almost negligible (Fig. 1C). Confocal microscopy studies of B cells incubated with anti-lgM-coated 1 µm and 3 µm beads revealed that some beads were totally phagocytosed (Fig. 1D, arrows), becoming inaccessible to the anti-goat IgG antibody, whereas others could still be detected outside (Fig. 1D, asterisks).

It was investigated whether phagocytosis of antigen by B2 B cells allows antigen presentation to T cells. As described above, the effect of RhoG-deficiency on B cell phagocytosis was stronger than that of RRas2 deficiency.. Therefore RhoG-deficient B cells were used for all subsequent functional studies. To evaluate if phagocytic B cells present antigen to T cells, purified naive B2 B cells from WT and Rhog-/- mice were preincubated with different ratios of beads coated with a mixture of anti-IgM and ovalbumin (OVA). Subsequently, these cells were incubated with purified Cell Trace Violet (CTV)-labeled T cells from OT-2 TCR transgenic mice for 3 days. OT-2 T cells respond to an OVA-derived peptide presented by I-Ab. Preincubation with beads coated with anti-IgM and OVA induced OT-2 T cell proliferation whereas preincubation with beads coated with either anti-IgM or OVA alone did not (Fig. 1E). These data suggest that B cell activation alone is not sufficient to activate CD4 T cells, but rather that OVA antigen uptake by B cells requires a BCR-dependent process. Furthermore, compared to WT B cells, proliferation of OT-2 T cells was reduced if B cells lacked RhoG (Fig. 1E Fig. 1E). Likewise, upregulation of IL-2Rα (CD25) expression by OT-2 T cells was antigen- and BCR triggering-dependent and mediated in part by RhoG (Fig. 1E). These data suggest that B cells take up antigen and present it in a dose-dependent manner to T cells by a phagocytic mechanism.

The GC reaction is regulated by a subset of CD4+ T cells (Tfh cells) that express the master regulator Bcl-6, as well as the surface markers CXCR5 and PD1. It was found that OT-2 T cells expressed CXCR5 and PD1 when stimulated with B cells that had been pre-incubated with 1 µm beads coated with anti-IgM and OVA in a RhoG-dependent process (Fig. 1G). To determine if OT-2 T cell differentiation to Tfh cells was also induced upon phagocytosis in an antigen-specific manner, B cells isolated from B1-8hi BCR knock-in mice were co-culture with OT-2 T cells. B1-8hi knock-in mice bear an already rearranged VDJ region in the IgH locus that in combination with a rearranging lambda light chain confers specificity for recognition of the hapten 4-hydroxy-3-nitrophenylacetyl (NP) and its iodinated derivative 4-hydroxy-3-iodo-5-nitrophenylacetic acid (NIP). It was found that OT-2 T cells proliferated and expressed CXCR5, PD1 and ICOS markers of Tfh cells in response to B1-8hi B cells that had phagocytosed 1 or 3 µm beads coated with NIP covalently bound to OVA carrier protein (NIP-OVA, Suppl. Fig. 1A and 1B). Interestingly, proliferation of OT-2 T cells increased with the dose of beads whereas expression of surface markers was optimum at intermediate doses of beads that depended on their diameter. This optimum was also observed for the generation of key Tfh cell cytokines involved in the GC response: IL-4, IL-6, and IL-21 (Fig. 2C). Interestingly, RhoG deficiency in B1-8hi B cells impaired cytokine release by OT-2 T cells (Fig. 2D), strongly suggesting that B cell phagocytosis of antigen is required. Altogether, the above data suggest that B cells can phagocytose antigen and present it to cognate T cells that become activated and adopt markers and properties of Tfh cells.

### Generation of GC B cells in vitro by a phagocytic-dependent mechanism.

In addition to promoting CD4+ T cell differentiation to Tfh cells, follicular B cells incubated with beads coated with anti-IgM and ovalbumin proliferated and expressed the GC B cell markers CD95 and GL7 (Fig. 3). They also upregulated CD40 and their proliferation depended on T cell help and RhoG expression (Fig. 3A, 3B and Fig. 4). These data suggested that antibody-mediated BCR triggering can promote the phagocytosis and presentation of antigen to T cells, inducing T cell and B cell proliferation as well as the acquisition of B cell and T cell markers typical of a GC response. To determine if GC B cell differentiation was induced upon phagocytosis in an antigen-specific manner, purified B1-8hi B cells were incubated with 1 µm beads coated with NIP-OVA in the presence of OT-2 T cells for 4 days. This led to the emergence of GC B cells characterized by the expression of the GL7 and CD95 markers and to B cell proliferation (Fig. 3C and 3D). Beads coated with NP linked to a different carrier protein (chicken gammaglobulin, CGG) did not elicit GC B cell differentiation or proliferation, indicating that T cell help is required. Likewise, emergence of a GC B cell phenotype was inhibited if B cells lacked RhoG, suggesting that beads, and the NIP-OVA antigen, were taken up by phagocytosis. A key feature of GC B cell differentiation is the expression of the master regulator Bcl-6. The emergence of a Bcl-6+ B cell population that also expresses the GL7 GC marker for 7 days of WT B cell culture were followed with NIP-OVA-coated 1 µm beads and OT-2 T cells. It was found a distinct double positive population that reached a maximum of 40% of all B cells at day 3 of co-culture (Fig. 3E). The maximum of Bcl-6+GL7+ B cells at day 3 was also coincident with the maximum for GL7+ B cells that had downregulated CD38, another feature of GC B cells (Fig. 4B). The analysis of Bcl-6 expression according to the number of cell divisions at day 3 showed that Bcl-6 upregulation peaked in B cells that had undergone 2 cell divisions and gradually decayed in cells that had undergone 3 divisions or more (Fig. 3F). B cells stimulated with NP-CGG-coated beads in the presence of OT-2 underwent a maximum of 3 cell divisions and did not upregulate Bcl-6 (Fig. 3F), indicating that T cell help was required. The bead-bound NB-CGG stimulus was also unable to upregulate the plasmacytic B cell master regulator Blimp-1 that occurred after the 3rd cell division (Fig. 3F). Bcl-6 and Blimp-1 are involved in a mutually regulatory loop in a way that Bcl-6 represses Blimp-1 expression and the latter represses Bcl-6, favoring the exit of cells from the GC differentiation program and terminal differentiation. Since Blimp-1 expression steadily increases with cell division the bell-shaped behavior of Bcl-6 expression with cell division could very well be originated by the growing repression exerted by Blimp-1 expression (Fig. 3G) and responsible for the decline in Bcl-6 expression after 3 days of culture (Fig. 3E). These results indicate that B cell stimulation with bead-bound antigen results in their differentiation to GC B cells in vitro that are regulated by Bcl-6 and Blimp-1 expression as it has been previously established in vivo.

In germinal centers, antigen-specific B cells form clusters of highly proliferating cells that segregate from non-responding B cells in follicles. In the culture plates in vitro, it was found the formation of large clusters containing as many as 4,000 cells when B cells were stimulated with 1 µm beads coated with anti-IgM plus ovalbumin (Fig. 5A). The clusters consisted of a mixture of tightly intermingled B cells and CD4+ T cells. Similar clusters were found when mixtures of NP-specific B1-8hi B cells and OT-2 T cells were incubated with 1 µm beads coated with NIP-OVA (Fig. 5B). Interestingly, stimulation of B cells with a similar dose (see below) of soluble NIP-OVA, resulted in the formation of much smaller clusters, suggesting that the large B cell and T cell aggregates were related to the phagocytic stimulus. Using mixtures of CTV-labeled B1-8hi B cells and CFSE-labeled OT-2 T cells, after 7 days of stimulation with NIP-OVA antigen-coated beads we found that the periphery of the cluster contained the cells with most diluted CTV and CFSE, suggesting that B and T cells proliferate and expand towards the edges of the clusters (Fig.5C). The periphery of the clusters also contained the highest percentage of B cells positive for the GC marker GL7 (Fig. 5D), suggesting that B cells proliferate and express GC markers towards the periphery. These data show that follicular B cells stimulated with antigen-coated beads form large clusters, together with T cells, that are reminiscent of germinal centers.

### A bead-bound but not a soluble antigen induces the generation of class-switched immunoglobulins of high affinity.

During the GC response, B cells recognize antigen through their BCR and this recognition has a dual effect: the activation of intracellular signaling pathways and antigen internalization for processing and presentation to CD4+ T cells. Thus, it was analyzed if the way in which the antigen is given to B cells (soluble versus phagocytic) influences B cell activation independently of antigen presentation to T cells. To normalize both stimuli for equal antigen presentation, a titration experiment in which proliferation of OVA-specific OT-2 CD4+ T cells was measured in response to B cells preincubated with different doses of soluble and bead-bound NIP-OVA were carried out. It was found that a concentration of 100 ng/ml soluble NIP-OVA was as effective as a dose of 3 NIP-OVA-coated beads per B cell for inducing OT-2 cell proliferation (Fig. 6A). Furthermore, those doses of stimuli were equally effective at inducing the expression of TFH cell markers in OT-2 T cells and T cell proliferation (Fig. 6B). In such conditions, B1-8hi B cells expressed GC B cell markers GL7 and CD95 independently of the soluble or bead-bound nature of the stimulus (Fig. 6C). By contrast, the form of antigen delivery to B cells (soluble vs. bead-bound) influenced B cell proliferation. Indeed, the soluble antigen was significantly less mitogenic than the bead-bound one (Fig. 6C). In addition to the different proliferation rate, bead-bound antigen was more effective at promoting expression of Bcl-6 and Blimp-1 than soluble antigen (Fig. 6D). On the contrary, soluble antigen was more effective at inducing the expression of the cytidine deaminase gene (Aicda) than bead-bound antigen. Nevertheless, bead- bound and soluble antigens were equally efficient at inducing somatic mutations in the nucleotide sequence, although the bead-bound stimulus was 3-fold more efficient than the soluble one at inducing changes in the amino acid sequence (Fig. 7A). These results suggest that B cells with V region mutations are being selected for certain amino acid mutations in our in vitro system. Indeed, most of the mutations mapped at, or in the proximity of, the antigen-binding CDR sequences (Fig. 7B). The effect of bead-bound antigen on upregulation of Aicda, Bcl-6 and Blimp1, and somatic mutation were related to a phagocytic process, for RhoG-deficient B cells were less competent in these processes than WT cells (Fig. 6D and Fig. 7D). These data indicate that a bead-bound, phagocytic-dependent antigen stimulus is more effective than soluble antigen at inducing B cell proliferation, as well as their differentiation into bona fide GC B cells. Indeed, bead-bound antigen was more effective than soluble antigen at inducing Ig class switch, which is a GC-dependent event, as evidenced by the expression of IgG1 at the B cell membrane (Fig. 6E). Furthermore, B cell stimulation with bead-bound antigen was a better inducer of the plasma cell differentiation marker CD138 than soluble antigen (Fig. 6F). In addition, we identified the CD138+ IgG1+ B cell plasmablast population only in samples stimulated with bead-bound antigen (Fig. 6G). These data suggest that stimulation of B cells with bead-bound antigen induces their differentiation into plasmablasts and antibody-secreting plasma cells.

The capacity of bead-bound antigen to induce plasma cell differentiation was paralleled by the detection of high affinity anti-NP Igs in 7-day culture supernatants. The anti-NP Igs were comprised of non-switched IgM but also of high amounts of class-switched IgG1, IgG2a, IgG3, IgA and lesser, but detectable, amounts of IgG2b (Fig. 8A). The production of high and low affinity class-switched anti-NP Igs by bead-bound antigen-stimulated cells was strongly inhibited if B cells lacked RhoG, suggesting that generation of high-affinity mature Igs required the beads to be phagocytosed. Likewise, the supernatant of WT B1-8hi B cell cultures stimulated with bead-bound antigen, but not with soluble antigen, contained high-affinity class-switched Igs (Fig. 8B). The presence of mature Igs was more evident at day 7 than at day 4 after stimulation with bead-bound antigen and included high concentrations of IgG1 but also IgG2a, IgG3 and IgA. The above experiments were carried out with B1-8hi B cells and OT-2 T cells purified by negative selection. To exclude the participation of a third cell type that could be contaminating the B and T cell populations, the experiments with bead-bound versus soluble NIP-OVA antigen were repeated with FACS-sorted follicular B (B220+CD23+CD43-CD11b-) and CD4+ OT-2 T cells (Fig. 9A). In these conditions, B cells acquired GC markers (Fig. 9B) and differentiated into antibody-producing cells able to secrete mature Igs (Fig. 9C), thus indicating that T and B cells are sufficient to generate the GC-like reaction. Overall, these data showed that incubation of naive B cells with a haptenated antigen immobilized onto 1 µm beads in the presence of antigen-specific helper CD4+ T cells in vitro results in the generation of high affinity antigen-specific antibodies of mature, class-switched, isotypes.

### B cell stimulation with bead-bound antigen generates functional antibodies from non-transgenic B cells in vitro.

To interrogate if the phagocytic-dependent antigen delivery to B cells can be used to generate antigen-specific antibodies in vitro out of a non-transgenic, BCR repertoire NP-OVA-coated 1 µm beads were incubated with purified B cells from non-transgenic C57BL/6 mice and with OT-2 T cells. After 7 days, the culture supernatant contained anti-NP IgMs but not detectable class-switched Igs (Fig. 8C). Since the concentrations of IL-4 and IL-21 in the culture supernatants of bead-bound antigen-stimulated B cells was low (Fig. 2C and 2D), to extend the life of the cultures by adding recombinant IL-4 and IL-21 at day 5 was attempted. It was found detectable anti-NP IgG1 and IgA in the cytokine-supplemented cultures after 10 days of incubation (Fig. 8C).

To determine if the in vitro system can be used to generate antibodies of medical interest, we coated 1 µm beads with Env recombinant protein of HIV and NIP-OVA as carrier protein. Coated beads were incubated with B cells from non-transgenic C57BL/6 mice and OT-2 T cells. We detected the generation of Env-specific IgMs both at day 7 and day 10 but not class-switched Igs (Fig. 8C). Nonetheless, the supernatant of 7-day cultures was tested for its capacity to inhibit the entry of HIV viral particles coated with the HIV Env protein or pseudotyped with the envelope protein of VSV. The supernatant inhibited in a dose-dependent manner the entry of the HIV Env-mediated virus but not of the VSV G-dependent one, suggesting that the generated anti-HIV Env IgMs specifically neutralize HIV.

### A bead-bound phagocytic stimulus provides a strong and sustained BCR signal.

To provide a mechanistic explanation to the above findings, it was interrogated if the bead-bound stimulus could result in a more intense or more sustained BCR signal than soluble antigen. The degree of occupancy of the BCR in both conditions using a fluorescent NP derivative were first determined. At the conditions used above for comparison (3 coated beads vs. 100 ng/ml of soluble protein), 35% of the B1-8 BCR was free to bind NP hapten in cells incubated with beads, whereas only 1% was free if cells had been incubated with soluble protein (Fig. 10A). BCR downregulation in function of time was also measured and found that the soluble stimulus was at least as effective as the bead-bound one at promoting BCR downregulation (Fig. 10B). These data indicate that the bead-bound antigen is not more effective than the soluble one in terms of BCR occupancy or BCR downregulation. Therefore, its superior capacity to produce class-switched high affinity antigen-specific Igs is not explained by simply higher BCR occupation. It was therefore investigated if signaling events downstream of the BCR were differentially activated. Phagocytosis requires the rearrangement of the actin cytoskeleton around the particle in the phagocytic cup. Thus, it was investigated if there were differences in terms of the intensity or duration of actin polymerization in B cells incubated with bead-bound vs. soluble antigen. Both stimuli equally increased polymerized F-actin levels in B cells after 1 minute of incubation (Fig. 10C). However, whereas the polymerization phase was rapidly followed by an intense depolymerization in B cells stimulated with soluble antigen, the high F-actin content was sustained in B cells stimulated with bead-bound antigen. The bead-bound stimulus elicited a more intense and sustained phosphorylation of Akt and ERK, which are two events linked to activation of the PI3K and Ras pathways, than the soluble stimulus (Fig. 10D). More importantly, phosphorylation of Syk, a direct BCR effector previously shown to mediate FcγR- and CR-dependent phagocytosis, was also more intense and sustained (Fig. 10D). This suggests that the bead-bound stimulus induces a stronger BCR signal that is more persistent in time than the soluble stimulus. To determine if the stronger signal promoted by the bead-bound stimulus was related to the phagocytic process, the phosphorylation of Akt and S6 (in the PI3K pathway) and of ERK in WT vs RhoG-deficient B cells was compared in response to bead-bound antigen. It was found that RhoG is required to induce and sustain those signals, as well as the phosphorylation of Syk and of the Igα subunit of the BCR, strongly suggesting that antigen phagocytosis elicits a longer and more intense BCR signal (Fig. 10E).

Next the cellular location of phosphorylated BCRs during antigen phagocytosis was assessed. Using fluorescent 1 µm beads and confocal microscopy, it was found that in B cells stimulated with bead-bound antigen for a short time (5 minutes), both phospho-Igα and phospho-Syk were only found in the phagocytic cups (Fig. 10F). Interestingly, both proteins were still found phosphorylated all around the phagocytosed beads at a late (30 minutes) time point. These results show that BCR phosphorylation persists in the intracellular phagosome and suggest that this might be the cause for sustained BCR signaling when antigen is taken up by a phagocytic mechanism.

### Phagocytosis of antigen by B cells induces a potent humoral response in vivo.

Once established that phagocytosis of antigen by B cells can drive the generation of a GC response and formation of mature high-affinity Igs, it was next interrogated if the process may also occur in vivo. If B cells phagocytosed beads in vivo was studied first. To this end, B1-8hi transgenic WT and Rhog-/- mice was inoculated with fluorescent 1 µm beads covalently bound to NIP-OVA by the intraperitoneal route and measure the presence of beads inside B cells in the spleen. To distinguish B cells that had phagocytosed beads entirely from B cells that had adherent beads, spleen B cells with a fluorescent anti-OVA antibody were extracellularly stained. Using controls of B cells incubated in vitro with beads on ice plus or minus anti-OVA (5AFig. 12A) the conditions for the in vivo experiment were set up. It was found that 5 hours after i.p. inoculation a measurable percentage of the NP+ spleen B cell population was detected with beads exclusively inside. The percentage of B cells with phagocytosed beads was 4-fold lower in RhoG-deficient mice than in their WT counterparts (Fig. 11). Phagocytosis by B cells unable to bind the NP hapten (NP-) was negligible, suggesting that the process was BCR-dependent. Both, MZ and follicular B1-8hi B cells phagocytosed NIP-OVA coated beads to a similar extend, and required the expression of RhoG and of the NP-specific BCR (Fig. 12B). Phagocytosis by NP+ B cells was higher than this of splenic CD11b+F4/80+ macrophages (0.14% vs. 0.05%), suggesting that the efficiency of phagocytosis by antigen-specific B cells was not lower than that of professional phagocytes (Fig. 12B and 12C).

. Here it is showed that the number of B cells with a GC phenotype in Peyer's patches of non-immunized mice or in the spleen of mice immunized with sheep red blood cells was not affected by RhoG deficiency (Fig. 13). Therefore, antigen phagocytosis mediated by RhoG does not seem to be required for the humoral T-dependent response to a soluble antibody or to erythrocytes. However, RhoG-deficient mice immunized by the intraperitoneal route with NIP-OVA covalently bound to 1 µm beads were less efficient (3-fold) in the generation of GC B cells (Fig. 11B). To determine if the defective GC response to bead-bound antigen of Rhog-/- mice was B cell intrinsic, purified B cells from WT and Rhog-/- mice (both expressing the CD45.2 allele) were adoptively transferred into WT CD45.1+ recipient mice and then immunized with bead-bound NIP-OVA as above. By gating on B cells bearing the CD45.2 vs. the CD45.1 marker, the effect of RhoG deficiency on the GC B cell response to bead-bound antigen could be assessed. It was found equal proportion of CD45.2+ WT and RhoG-deficient B cells in both sets of mice, indicating that RhoG deficiency did not affect migration to the spleen of B cells or their survival (Fig. 11C). However, the percentage of B cells with GC markers within the transferred population (CD45.2+) was drastically reduced suggesting that B cells required to phagocytose the bead-bound antigen to become GC B cells. The difference in the percentage of GC B cells within the endogenous WT B cell population (CD45.1+) in both groups of mice was not significant. The above results show that B cells can phagocytose antigen in vivo and that by doing so enter into the GC reaction.

The use of beads that can be phagocytosed by B cells is not yet implemented as a mechanism to boost or modulate the humoral response. However, it is well known that in order to elicit a protective humoral response, vaccines need to incorporate an adjuvant that most frequently consists of aluminum compounds known as "alum". Interestingly, antigen becomes trapped within large 1-10 µm aggregates formed by alum which are thought to favor phagocytosis by dendritic cells. Using a combination of NIP-OVA plus alum for vaccination, a stronger production of high affinity class-switched Igs in WT mice compared to the immunization with soluble NIP-OVA antigen was detected (Fig. 11D). Interestingly, although RhoG deficiency did not impair the response to soluble antigen, it blocked the boosting effect of the alum adjuvant detected in the generation of anti-NP antibodies of the IgG2b and IgG3 subclasses. These results suggest that the RhoG-dependent phagocytosis mechanism is involved in the response to antigen plus alum immunizations. We additionally performed adoptive transfer experiments to study whether RhoG was necessary for GC formation and Ig class switch in a B cell-intrinsic manner. Purified B cells from WT or Rhog-/- mice (CD45.2+) were inoculated into CD45.1+ wild type (WT) receptor mice and subsequently were immunized with NIP-OVA plus alum. The GC response in the Rhog-/- CD45.2+ population was strongly inhibited in terms of CD95 and GL7 GC marker expression, expansion of NP-binding B cells or expression of class-switched IgG1, compared to WT B cells (Fig. 14A). These results suggest that antigen phagocytosis by B cells is required for a GC response to immunization with antigen plus alum.

To assess the effect of RhoG deficiency in B cells on antibody production, adoptive transfer experiments in Rag1-/- mice which lack endogenous T and B cells were carried out. These mice were reconstituted with purified WT OT-2 T cells and either with purified WT or with purified RhoG-deficient B cells. Subsequently, they were immunized with NIP-OVA either soluble or complexed with alum. The presence of low and high affinity anti-NP antibodies was evaluated to find that mice reconstituted with WT B cells produced low and high affinity IgM and class-switched Igs, whereas mice reconstituted with RhoG-deficient B cells were incompetent to produce class-switched anti-NP immunoglobulins of high affinity (Fig. 11E and Fig. 14). These results suggest that phagocytosis of antigen by B cells might be involved in the in vivo generation of high affinity mature immunoglobulins in response to antigen plus alum formulation.

## Claims

1. A method for the *in vitro* generation of antigen-specific antibodies or cells producing thereof, said method comprising:
a) culturing B cells with an antigen-coated carrier for at least 3 days, wherein said antigen-coated carrier has a size between 0.5 µm and 20 µm, and
b) co-culturing the B cells obtained from step a) with CD4+ T cells for at least 3 days.

2. The method according to claim 1, wherein the B-cells from step a) are naive B cells expressing non-class-switched immunoglobulins or memory B cells expressing class-switched immunoglobulins.

3. The method according to claim 1 or 2, wherein the CD4⁺ T cells of step b) are naive CD62L⁺, CD4⁺ T cells or memory CD44⁺, CD4⁺ T cells.

4. The method according to any one of claims 1 to 3, wherein step a) and b) are simultaneously executed.

5. The method according to any one of claims 1 to 4, wherein said method is further **characterized by** the absence in the culture of step a) of cells with antigen presenting capacity other than the B cells.

6. The method according to any one of claims 1 to 5, wherein the size of the antigen-coated carrier is 1, 2, 3, 4, 5, 6, 7, 8, 9, 10, 11, 12, 13, 14, 15, 16, 17, 18 or 19 µm.

7. The method according to any one of claims 1 to 6, where the antigen-coated carrier from step a) is an aggregate of small particles or a magnetic particle, preferably, a paramagnetic particle.

8. The method according to any one of claims 1 to 7, wherein said B cells are follicular B cells comprising low expression levels of CD21, high expression levels of CD23, and CD43 negative.

9. The method according to any one of claims 1 to 8, wherein the ratio of B cells to CD4+ T cells in step b) is 1:1.

10. The method according to any one of claims 1 to 9, wherein the cells of step a) are human cells.

11. The method according to any one of claims 1 to 10, wherein said antigen is selected from the group consisting of an hapten, peptide and protein, or a fragment of any thereof, preferably, the protein is a glycoprotein or a lipoprotein.

12. The method according to any one of claims 1 to 11, wherein said antigen is derived from a pathogen, preferably selected from the group consisting of virus, bacteria, yeast and protozoa.

13. The method according to any one of claims 1 to 12, wherein said method results in the production of high affinity class switched antibodies, wherein high affinity antibodies are **characterized by** binding to their antigen with a dissociation constant (KD) of 10⁻⁵ to 10⁻¹² moles/liter or less.

14. The method according to any of claims 1 to 13, wherein said method further comprises:
c) selecting cells producing high affinity antigen-specific antibodies; and
d) optionally, before or after the selection step in c) isolating and/or immortalizing said cells.

15. The method according to claim 14, wherein the cells selected in step c) are producing high affinity class switched antibodies, preferably selected from the group consisting of IgG and IgA isotypes, more preferably selected from the group consisting of IgG1, IgG2a, IgG3 and IgA isotypes .

16. A high affinity class switched antibody obtained by a method according to any one of claims 1 to 15, preferably, the high affinity antibody is **characterized by** binding to their antigen with a dissociation constant (KD) of 10-5 to 10-12 moles/L or less.


SEQUENCE LISTING

<110>  CONSEJO SUPERIOR DE INVESTIGACIONES CIENTÍFICAS (CSIC)

<120>  IN VITRO PRODUCTION OF HIGH AFFINITY MONOCLONAL ANTIBODIES

<130>  EP1641.1287

<160>  15

<170>  PatentIn version 3.5

<210>  1
<211>  24
<212>  DNA
<213>  Artificial Sequence

<220>
<223>  Forward primer for amplifying B1-8Vh gene

<400>  1
ccatgggatg gagctgtatc atcc                                              24


<210>  2
<211>  23
<212>  DNA
<213>  Artificial Sequence

<220>
<223>  Reverse primer for amplifying B1-8Vh

<400>  2
gaggagactg tgagagtggt gcc                                               23


<210>  3
<211>  20
<212>  DNA
<213>  Artificial Sequence

<220>
<223>  Forward primer for Bcl6

<400>  3
ggaagttcat caaggccagt                                                   20


<210>  4
<211>  18
<212>  DNA
<213>  Artificial Sequence

<220>
<223>  Reverse primer for Bcl6

<400>  4
gacctcggta ggccatga                                                     18


<210>  5
<211>  19
<212>  DNA

<213>  Artificial Sequence

<220>
<223>  Forward primer for Bcl2

<400>  5
gtacctgaac cggcatctg                                                    19


<210>  6
<211>  20
<212>  DNA
<213>  Artificial Sequence

<220>
<223>  Reverse primer for Bcl2

<400>  6
ggggccatat agttccacaa                                                   20


<210>  7
<211>  21
<212>  DNA
<213>  Artificial Sequence

<220>
<223>  Forward primer for Blimp1 gene

<400>  7
ggctccacta cccttatcct g                                                 21


<210>  8
<211>  19
<212>  DNA
<213>  Artificial Sequence

<220>
<223>  Reverse primer for Blimp1 gene

<400>  8
tttgggttgc tttccgttt                                                    19


<210>  9
<211>  20
<212>  DNA
<213>  Artificial Sequence

<220>
<223>  Forward primer for Irf4 gene

<400>  9
ggagtttcca gaccctcaga                                                   20


<210>  10
<211>  20
<212>  DNA
<213>  Artificial Sequence

<220>

<223>  Reverse primer for Irf4 gene

<400>  10
ctggctagca gaggttccac                                                    20


<210>  11
<211>  19
<212>  DNA
<213>  Artificial Sequence

<220>
<223>  Forward primer for HPRT gene

<400>  11
tcctcctcag caagctttt                                                     19


<210>  12
<211>  21
<212>  DNA
<213>  Artificial Sequence

<220>
<223>  Reverse primer for HPRT gene

<400>  12
cctggttcat catcgctaat c                                                  21


<210>  13
<211>  20
<212>  DNA
<213>  Artificial Sequence

<220>
<223>  Forward primer for GAPDH gene

<400>  13
ctcccactct tccaccttcg                                                    20


<210>  14
<211>  24
<212>  DNA
<213>  Artificial Sequence

<220>
<223>  Reverse primer for GAPDH gene

<400>  14
cataccagga aatgagcttg acaa                                               24


<210>  15
<211>  134
<212>  PRT
<213>  Artificial Sequence

<220>
<223>  Amino acid sequence of B1-8Vh

<400>  15

Gly Leu Asp Ile Tyr Met Gly Asp Asn Asp Ile His Phe Ala Phe Leu
1               5                   10                  15

Ser Thr Gly Val His Ser Gln Val Gln Leu Gln Gln Pro Gly Ala Glu
            20                  25                  30

Leu Val Lys Pro Gly Ala Ser Val Lys Leu Ser Cys Lys Ala Ser Gly
        35                  40                  45

Tyr Thr Phe Thr Ser Tyr Leu Met His Trp Val Lys Gln Arg Pro Gly
    50                  55                  60

Arg Gly Leu Glu Trp Ile Gly Arg Ile Asp Pro Asn Ser Gly Gly Thr
65                  70                  75                  80

Lys Tyr Asn Glu Lys Phe Lys Ser Lys Ala Thr Leu Thr Val Asp Lys
                85                  90                  95

Pro Ser Ser Thr Ala Tyr Met Gln Leu Ser Ser Leu Thr Ser Glu Asp
            100                 105                 110

Ser Ala Val Tyr Tyr Cys Ala Arg Tyr Asp Tyr Tyr Gly Ser Ser Tyr
        115                 120                 125

Phe Asp Tyr Trp Gly Gln
    130